# EUROPEAN PATENT APPLICATION

(11) **EP 4 520 831 A1**
(43) Date of publication of application: **12.03.2025**
(21) Application number: 23195542.8
(22) Date of filing: 05.09.2023
(51) Int. Cl.: C12N 15/86, A61K 48/00, A61K 38/37, C07K 14/755

(54) **NOVEL LENTIVIRAL VECTOR PARTICLES**

(71) Applicant: Fondazione Telethon ETS, 00185 Roma (IT); Ospedale San Raffaele S.r.l., 20132 Milano (IT); Università degli Studi del Piemonte Orientale "Amedeo Avogadro", 13100 Vercelli (IT)
(72) Inventor: MILANI, Michela, Milan (IT); CANTORE, Alessio, Milan (IT); FOLLENZI, Antonia, Novara (IT)
(74) Representative: Sagittarius IP

(57) **Abstract**

The present invention relates to a lentiviral vector particle pseudotyped with the baculovirus-derived GP64 envelope protein for the transduction of liver endothelial cells. Provided are methods of liver endothelial cell transduction *in vitro, in vivo* and *ex vivo,* wherein a nucleic acid is transduced by means of the GP64 pseudotyped lentiviral vector particle. Also provided is a method of treating a subject in need of liver regeneration or suffering from a liver disease or haemophilia comprising administration of a GP64 pseudotyped lentiviral vector particle, and the GP64 pseudotyped lentiviral vector particle for use in transducing liver endothelial cells *in vivo,* such as for liver regeneration or for the treatment of a liver disease or haemophilia. Still further provided is a GP64 pseudotyped lentivirus vector particle comprising a nucleic acid, said nucleic acid comprising the long or short form endogenous human factor VIII (F8) promoter operably linked to a coding sequence.

## Description

### FIELD OF THE INVENTION

The present invention relates to a lentiviral vector particle pseudotyped with the baculovirus-derived GP64 envelope protein for the transduction of liver endothelial cells. Provided are methods of liver endothelial cell transduction *in vitro, in vivo* and ex *vivo,* wherein a nucleic acid is transduced by means of the GP64 pseudotyped lentiviral vector particle. Also provided is a method of treating a subject in need of liver regeneration or suffering from a liver disease or haemophilia comprising administration of a GP64 pseudotyped lentiviral vector particle, and the GP64 pseudotyped lentiviral vector particle for use in transducing liver endothelial cells *in vivo,* such as for liver regeneration or for the treatment of a liver disease or haemophilia. Still further provided is a GP64 pseudotyped lentivirus vector particle comprising a nucleic acid, said nucleic acid comprising the long or short form endogenous human factor VIII (F8) promoter operably linked to a coding sequence.

### BACKGROUND OF THE INVENTION

The liver is one of the most relevant target organs for *in vivo* gene therapy, since it offers the possibility to treat several inherited metabolic disorders, including plasma protein deficiencies, such as haemophilia (Cantore et al. (2022) Front. Med., 8:774618, doi: https://doi.org/10.3389/fmed.2021.774618; and Zabaleta et al. (2019) Hum. Gene. Ther., 30(1):1190-1203, doi: https://doi.org/10.1089/hum.2019.140). Adeno-associated virus (AAV) derived vectors and lentiviral vectors (LV) are both efficient vehicles for *in vivo* gene transfer to the liver, with the former being in advanced phase of clinical development or commercially available, while the latter have yet to reach clinical testing in the context of systemic administration (Cantore & Naldini (2021) Haemophilia, 27(S3):122-125, doi: https://doi.org/ 10.1111/hae.14056). A single intravenous (i.v.) administration of AAV vectors delivering a functional clotting factor transgene showed prolonged therapeutic benefit in adult people with haemophilia (Mahlangu et al. (2023) NEJM, 388:694-705, doi: 10.1056/NEJMoa2211075; and Pipe et al. (2023) NEJM, 388:706-718, doi: 10.1056/NEJMoa2211644). Furthermore, systemic administration of lentiviral vectors (LV) has showed stable liver gene transfer in animal models, such as mice, dogs and non-human primates, and allowed phenotypic correction of haemophilia models (Cantore et al. (2015), Sci. Transl. Med., 7(277):277ra28, doi: https://doi.ora/10.1126/scitranslmed.aaa1405; Merlin et al. (2017) Mol. Ther., 25(8):1815-1830, doi: https://doi.org/10.1016/i.vmthe.2017.04.029; Milani et al. (2019) Sci. Transl. Med., 11(493):eaav7325, doi: https://doi.ora/10.1126/scitranslmed.aav7325; Milani et al. (2022) Nat. Comm., 13:2454, doi: https://doi.org/10.1038/s41467-022-30102-3; and Nicolas et al. (2022) Nat. Commun., 3(1):5012, doi: https://doi.org/10.1038/s41467-022-32576-7). Compared to AAV vectors, LV integrate into the genome of transduced cells, potentially ensuring life-long gene transfer even after a single administration to paediatric patients and possess larger packaging capacity. Moreover, the percentage of humans with anti-AAV neutralizing antibodies, which are generally excluded from receiving systemic AAV-vector gene therapy is higher than the prevalence of anti-LV immunity. For these reasons, LV may complement the reach of AAV-vector mediated liver gene therapy in the future.

Typically, the tropism of AAV vectors depends on the capsid serotype, while that of LV is directed by the envelope protein. The surface glycoprotein of the vesicular stomatitis virus (VSV.G) is the most widely used envelope protein to pseudotype LV, as it confers broad tropism and high stability to LV particles. However, the high fusogenicity of VSV.G causes rapid syncytia formation and cell death in stable VSV.G-expressing cell lines (used to package and produce VSV.G pseudotyped virus; Ory et al. (1996) Proc. Natl. Acad. Sci. USA, 93(21):11400-11406, doi: https://doi.org/10.1073%2Fpnas.93.21.11400), and its broad tropism can lead to off target effects caused by the transduction of cells other than those of interest. For example, VSV.G-pseudotyped LV (VSV.G-LV) can efficiently transduce both mouse and human antigen presenting cells (APCs), eliciting an immune response to the transgene product being transduced and thus negating any therapeutic effects (Dyall et al. (2007) Blood, 97(1):114-121, doi: https://doi.org/10.1182/blood.v97.1.114; Chinnasamy et al. (2000) Hum. Gene Ther., 11(13):1901-1909, doi: https://doi.org/10.1089/ 10430340050129512; Metharom et al. (2001) Hum. Gene Ther., 12(18):2203-2213, doi: https://doi.ora/10.1089/10430340152710540; and VandenDriessche et al. (2002) Blood, 100(3):813-822, doi: https://doi.org/10.1182/blood.v100.3.813). Therefore, numerous alternative LV pseudotypes have been investigated (Frank & Buchholz (2018) Mol. Ther. Methods Clin. Dev., 12:19-31, doi: https://doi.ora/10.1016/i.omtm.2018.10.006; and Gutierrez-Guerrero et al. (2020) Viruses, 12(9):1016, doi: https://doi.org/10.3390/v12091016). The present inventors have previously shown that, upon i.v. administration, most VSV.G-LV distribute to the liver, where they transduce hepatocytes, liver macrophages (Kupffer cells, KC) and liver sinusoidal endothelial cells (LSEC; Merlin *et al.* (2017); Milani *et al.* (2019); and Merlin et al. (2019) Blood Adv., 3(5):825-838, doi: https://doi.org/10.1182/ bloodadvances.2018027979). All these cell types are important targets for different therapeutic purposes, in particular, hepatocytes naturally produce coagulation factor IX (FIX), which is lacking in haemophilia B, while coagulation factor VIII (FVIII), whose lack causes haemophilia A, is physiologically produced by LSEC, among other sources (Follenzi et al. (2008) J Clin Invest., 118(3):935-945, doi: https://doi.org/10.1172/JCI32748; and Zanolini et al. (2015) Haematologica, 100(7):881-892, doi: https://doi.org/10.3324/ haematol.2014.123117).

There therefore exists a need to develop LVs which achieve efficient gene transfer, in particular *in vivo,* with a more restricted tropism compared to VSV.G pseudotyped LVs, especially for the delivery of nucleic acids to the liver. Such LVs will find great utility in gene therapy and liver-directed gene therapy, in particular for the treatment of haemophilia.

### SUMMARY OF THE INVENTION

According to a first aspect of the invention, there is provided a method of transducing liver endothelial cells with a nucleic acid, wherein the nucleic acid is provided with a lentiviral vector particle pseudotyped with the baculovirus-derived GP64 envelope protein.

According to a further aspect, there is provided a method of treating a subject in need of liver regeneration or suffering from a liver disease or haemophilia, the method comprising administering to the subject a lentiviral vector particle pseudotyped with the baculovirus-derived GP64 envelope protein and comprising a nucleic acid as described herein.

In a yet further aspect of the invention, there is provided a lentiviral vector particle pseudotyped with the baculovirus-derived GP64 envelope protein for use in the transduction of liver endothelial cells with a nucleic acid *in vivo.*

According to a still further aspect, there is provided a lentivirus vector particle pseudotyped with the baculovirus-derived GP64 envelope protein and comprising a nucleic acid, said nucleic acid comprising the long or short form endogenous human factor VIII (F8) promoter operably linked to a coding sequence.

In some embodiments, transduction is for liver regeneration or for the treatment of a liver disease or haemophilia. Thus, in further embodiments the lentiviral vector particle comprises a nucleic acid comprising a coding sequence for a gene for liver regeneration, or a gene defective or deficient in a liver disease or haemophilia. In still further embodiments, the nucleic acid comprises a liver-specific promoter operably linked to a coding sequence.

In certain embodiments, the liver endothelial cells are liver sinusoidal endothelial cells (LSECs) and/or LSECs are transduced. In other particular embodiments, the lentiviral vector particle further comprises high levels of CD47, or a functional fragment thereof, on its surface.

### BRIEF DESCRIPTION OF THE FIGURES

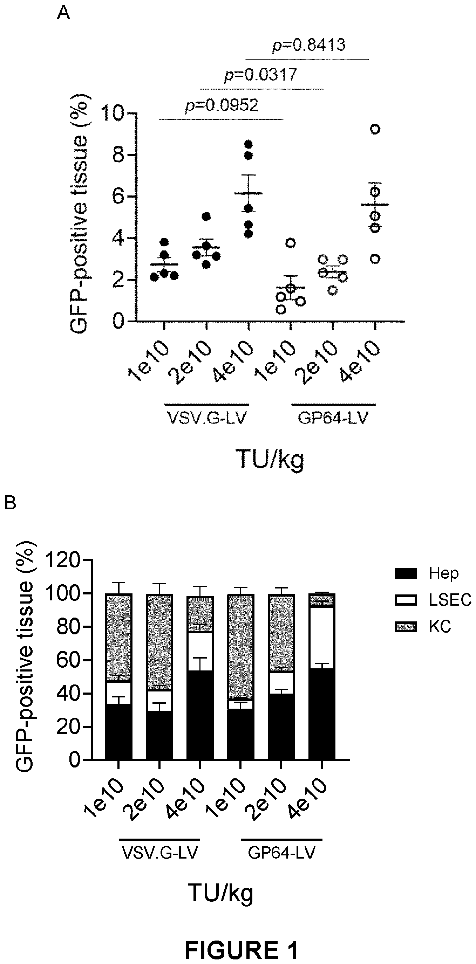

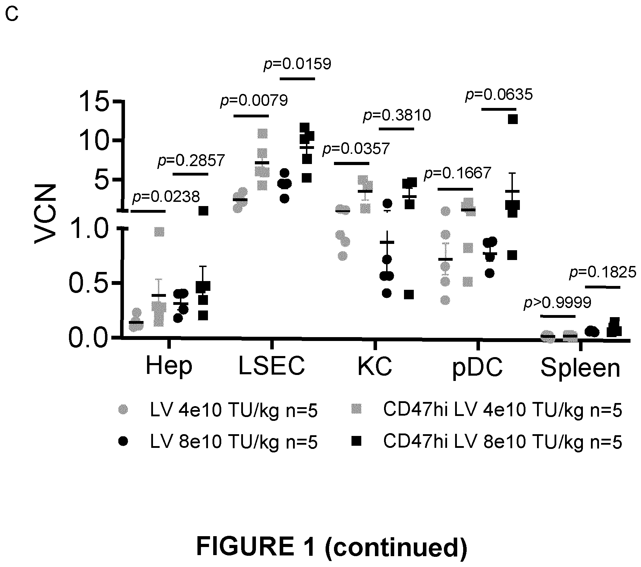

**Figure 1****: GP64-LV Efficiently Transduce Liver Sinusoidal Endothelial Cells *In Vivo.* (A)** Mean and standard error of the mean (SEM) with single values of the GFP-positive tissue measured in the liver of 8-week old mice 1 week after intravenous (i.v.) administration of VSV.G-pseudotyped (VSV.G-LV) or GP64-pseudotyped (GP64-LV) LV at the indicated doses (n=5 mice per group). Mann Whitney test. **(B)** Mean and SEM of the distribution of the GFP-positive tissue among the major liver cell types of mice shown in A at the indicated doses (KC: Kupffer cells; LSEC: liver sinusoidal endothelial cells; Hep: hepatocytes). **(C)** Mean with SEM with single values of vector copies per diploid genome (vector copy number, VCN) measured in FACS-sorted Hep, LSEC, KC, plasmacytoid dendritic cells (pDC), and whole spleen, as indicated, of NOD mice (n=5), 2 months after administration of the indicated doses of LV or phagocytosis-shielded CD47-high LV (CD47hi LV). Mann-Whitney test.

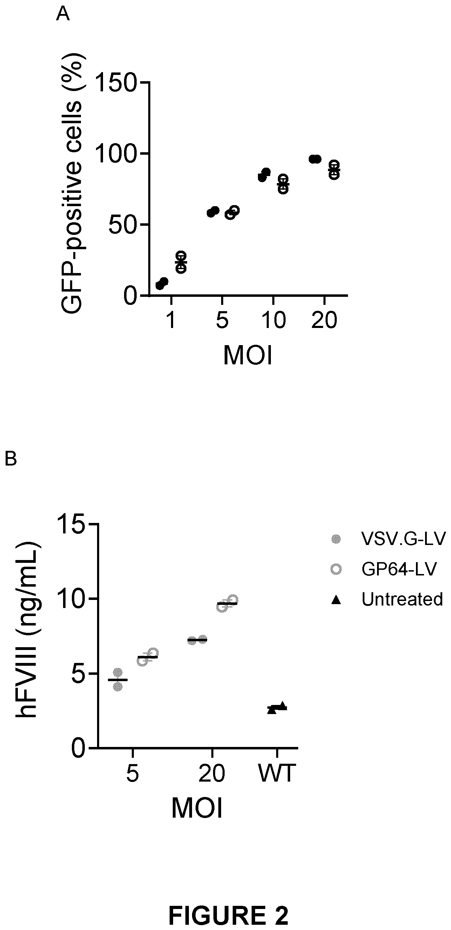

**Figure 2****: *Ex Vivo* Transduction of Primary Human Immortalized LSEC. (A)** Mean and SEM with single values of the % of GFP-positive primary human immortalized LSEC derived from a healthy donor (n=2 independent experiments) transduced with VSV.G-LV (closed circles) or GP64-LV (open circles) at the indicated dose (multiplicity of infection, MOI). (B) Mean and SEM with single values of the amount of human coagulation factor VIII (hFVIII) measured in the culture supernatant of primary human immortalized LSEC derived from a healthy donor (n=2 independent experiments) transduced with VSV.G-LV or GP64-LV at the indicated dose, or left untreated.

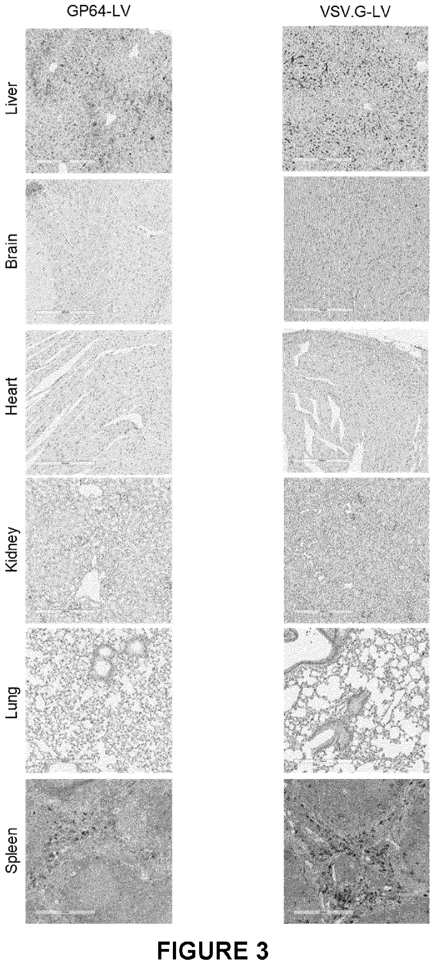

**Figure 3****: Biodistribution Analysis of GP64-LV or VSV.G-LV after Systemic Administration in Mice.** Representative immunohistochemistry images of the indicated organs stained with a primary anti-GFP antibody of mice treated at 8 weeks of age with 4×10¹⁰ TU/kg of GP64-LV or VSV.G-LV to provide a GFP transgene, as indicated, and analysed 10 days after i.v. LV administration (n=6 mice per group).

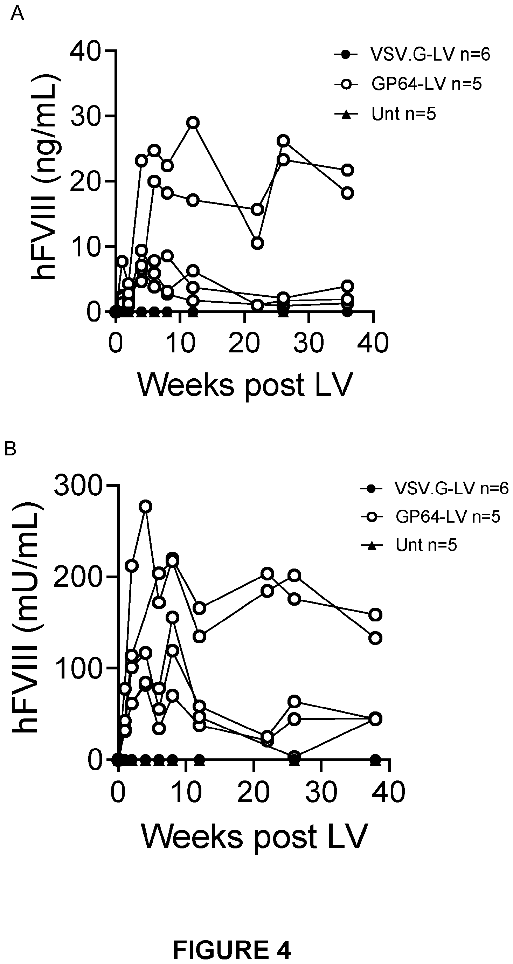

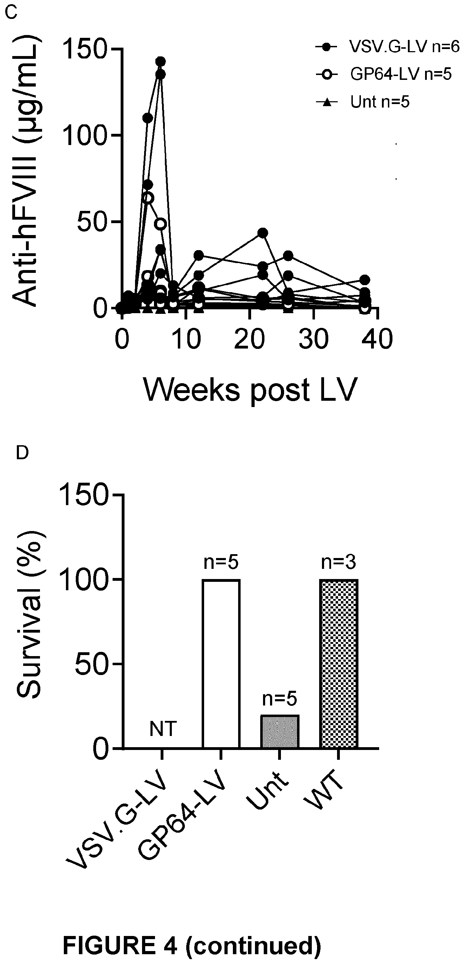

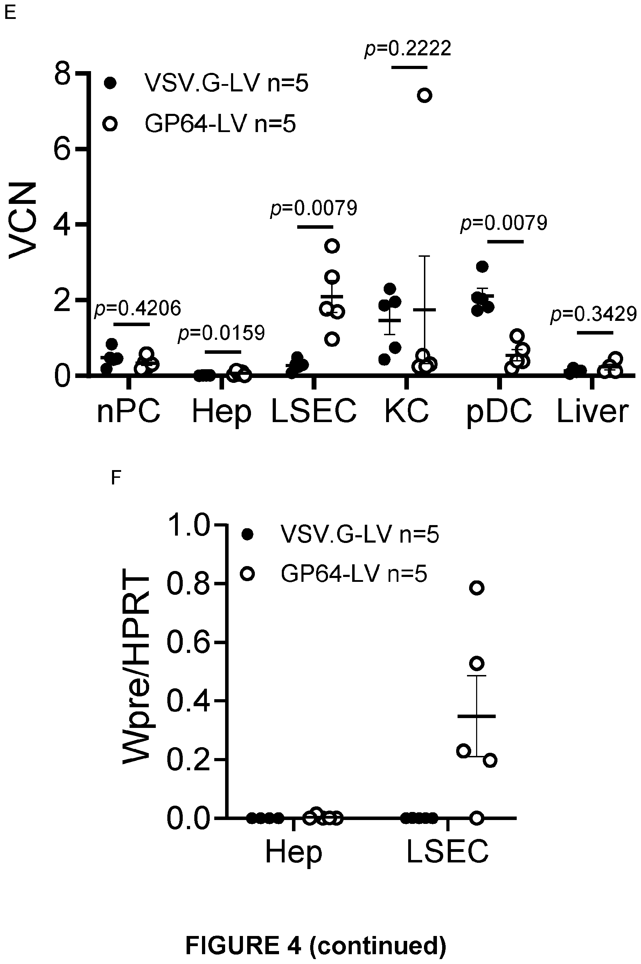

**Figure 4****: Evaluation of GP64-LV Mediated Gene Therapy in Haemophilia A Mice. (A-C)** Single values of hFVIII antigen **(A),** activity **(B)** or anti-hFVIII antibodies (Abs, **C)** measured over time in the plasma of immune competent HemoA-R593C mice treated at 7 weeks of age by i.v. injection of 5×10¹⁰ TU/kg of VSV.G-LV (n=6) or GP64-LV (n=5) expressing hFVIII under the control of the endogenous F8 promoter or left untreated (n=5). **(D)** Survival of mice shown in **A-C** 24 hours after haemostatic challenge. Note that mice treated with VSV.G-LV were not tested (NT) since they do not display measurable hFVIII in the circulation. WT: wild type. **(E)** Mean with SEM with single values of VCN measured in non-parenchymal cells (nPC), FACS-sorted Hep, LSEC, KC, pDC and whole liver, as indicated, of mice shown in **A-C,** 36 weeks after LV administration. Mann-Whitney test. **(F)** Mean with SEM with single values of LV expression measured in FACS-sorted Hep or LSEC, as indicated, of mice shown in **A-C,** 36 weeks after LV administration.

### DETAILED DESCRIPTION OF THE INVENTION

The present invention is based on the finding that a lentiviral vector particle (LV) pseudotyped with the baculovirus-derived GP64 envelope protein (GP64-LV) displays good transduction of liver endothelial cells *in vivo* and liver endothelial cells (LSECs) in particular, with improved tropism for these cells and other endothelial cells compared to a LV pseudotyped with the commonly used surface glycoprotein of vesicular stomatitis virus (VSV.G). GP64-pseudotyping has previously been shown to confer tropism restriction against haematopoietic lineage cells *in vitro* and improved resistance to complement-mediated inactivation (Schauber et al. (2004) Gene Therapy, 11:266-275, doi: https://doi.org/10.1038/sj.at.3302170; and Schauber-Plewa et al. (2005) Gene Therapy, 12(3):238-245, doi: https://doi.org/ 10.1038/sj.gt.3302399), properties important for efficient gene transfer/delivery. However, demonstrated herein in particular is the novel finding that GP64-LV transduces LSECs at a higher efficiency *in vivo* compared to VSV.G pseudotyped lentiviral vector (VSV.G-LV), together with confirmation that GP64-LV displays higher resistance to complement-mediated inactivation.

Thus, in a first aspect of the invention there is provided a method of transducing liver endothelial cells with a nucleic acid, wherein the nucleic acid is transduced by means of a lentiviral vector particle pseudotyped with the baculovirus-derived GP64 envelope protein.

The terms "transduction", "transducing" and the like as used herein refer to the process of introducing 'foreign' or exogenous nucleic acid into a cell using a virus or viral vector. Thus, as used herein "transduction" is viral transduction and the terms "transducing", "introducing", "delivering", "providing" and the like may be used interchangeably herein. The nucleic acid may be DNA or RNA (e.g. mRNA) encoding a gene of interest, such as an exogenous sequence which has no endogenous equivalent in the cell being transduced or an exogenous sequence which is substantially the same as a sequence which is already present in the cell being transduced (i.e. an endogenous sequence), either in the genome or otherwise contained in the cell which may or may not be expressed. The exogenous sequence may be the same as an endogenous sequence, e.g. it can be considered a replacement of an endogenous sequence/gene such as wherein said endogenous sequence/gene contains an inactivating or otherwise deleterious mutation or is not expressed by the transduced cell (e.g. it is silenced in the transduced cell type), or the exogenous sequence may differ from the endogenous sequence/gene, e.g. it may contain an activating mutation wherein the endogenous sequence/gene is inactive in the transduced cell. Alternatively, the nucleic acid may be non-coding, such as without limitation micro RNA (miRNA), silencing RNA (siRNA), piwi-interacting RNA (piRNA), repeat associated small interfering RNA (rasiRNA), transfer RNA (tRNA), ribosomal RNA (rRNA), small nuclear RNA (snRNA), small nucleolar RNA (snoRNA) and long ncRNA (IncRNA). Certain non-coding nucleic acids (e.g. miRNAs and siRNAs) are useful for the inhibition or downregulation of expression of an endogenous gene in the transduced cell, such as wherein the regulation of expression of said gene is dysregulated.

### Viral (e.g. Lentiviral) Vectors

A Vector is a tool that provides or facilitates the transfer of an entity from one environment to another, i.e. they are used to deliver an entity (e.g. a nucleic acid). In the present invention, the vectors used in the method of transducing liver endothelial cells are viral vectors. In one embodiment, the viral vectors are retroviral vectors. In a preferred embodiment, the vectors are lentiviral vectors.

A retroviral vector may be derived from any suitable retrovirus. For example, any of the large number of retroviruses identified to date may be used, including for example murine leukaemia virus (MLV), human T cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) or avian erythroblastosis virus (AEV). Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses.

Lentiviruses make up a genus of retroviruses that can cause chronic diseases characterised by long incubation/latency periods. The are able to infect mammalian cells, affecting humans and other mammals including apes, horses, cows, sheep and other ruminants. A particular example of lentivirus is human immunodeficiency virus (HIV). Thus, in sone embodiments the lentiviral vectors are derived from an immunodeficiency virus, such as HIV-1, HIV-2, SIV, FIV, BIV, EIAV, CAEV or visna lentivirus. In a further embodiment, the viral vector is a gamma-retroviral vector. Lentiviruses are able to integrate a significant amount of viral DNA into the genome of the infected/host cell, becoming endogenous by way of their genome becoming integrated into the host germline genome meaning the viral sequence is inherited. This viral DNA comprises a 5' LTR and a 3' LTR, between/within which are located a packaging signal to enable the genome to be packaged, a primer binding site, integration sites to enable integration into a host cell genome, and *gag, pol* and *env* genes encoding the packaging components. Lentiviruses have additional features, such as *rev* and RRE sequences in HIV, which enable the efficient export of RNA transcripts of the integrated provirus from the nucleus to the cytoplasm of an infected target cell. In the provirus, these genes are flanked at both ends by regions called long terminal repeats (LTRs). The LTRs are responsible for proviral integration and transcription. LTRs also serve as enhancer-promoter sequences and can control the expression of the viral genes. The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses. In a defective retroviral vector genome gag, *pol* and *env* may be absent or not functional. In a typical retroviral vector, at least part of one or more protein coding regions essential for replication may be removed from the virus. This makes the viral vector replication deficient. Lentiviruses can also effectively infect both dividing and non-dividing/slowly dividing cells, and display low immunogenicity. As such, they are effective tools for gene delivery and therapy. As retroviruses, lentiviruses have the viral protein-coding genes *gag*, *pol,* and *env* in their RNA genome, in the order 5'*-gag-pol-*env-3'. However, lentiviruses have two further genes which have regulatory functions, *tat* and *rev.* As RNA viruses, they carry specific proteins within their capsids which associate with the RNA genome and are involved in the early stages of viral genome replication, e.g. reverse transcriptase which is the virally encoded RNA-dependent DNA polymerase and integrase which binds the viral cDNA generated by reverse transcriptase and the host cell's DNA, processing the LTR sequences before inserting the viral cDNA into the host DNA. *Tat* acts as a trans-activator during transcription to enhance initiation and elongation and the *Rev* responsive element acts post-transcriptionally, regulating mRNA splicing and transport to the cytoplasm. A lentiviral vector, as used herein, is a vector which comprises at least one component part derivable from a lentivirus. Preferably, that component part is involved in the biological mechanisms by which the vector infects cells, expresses genes or is replicated.

As examples of lentivirus-based vectors, HIV-1- and HIV-2-based vectors are described. The HIV-1 vector contains cis-acting elements that are also found in simple retroviruses. It has been shown that sequences that extend into the gag open reading frame are important for the packaging of HIV-1. Therefore, HIV-1 vectors often contain the relevant portion of gag in which the translational initiation codon has been mutated. In addition, most HIV-1 vectors also contain a portion of the *env* gene that includes the *RRE.* Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat. Most HIV-2-based vectors are structurally very similar to HIV-1 vectors. Similar to HIV-1-based vectors, HIV-2 vectors also require RRE for efficient transport of the full-length or singly spliced viral RNAs. In one system, the vector and helper constructs are from two different viruses, and the reduced nucleotide homology may decrease the probability of recombination. In addition to vectors based on the primate lentiviruses, vectors based on FIV have also been developed as an alternative to vectors derived from the pathogenic HIV-1 genome. The structures of these vectors are also similar to the HIV-1 based vectors. The viral vector may have a minimal viral genome. By "minimal viral genome" it is to be understood that the viral vector has been manipulated so as to remove the non-essential elements and to retain the essential elements in order to provide the required functionality to infect, transduce and deliver a nucleic acid of interest to a target cell. A plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. The vector may lack a functional *gag-pol* and/or *env* gene and/or other genes essential for replication. A plasmid vector used to produce the viral genome within a host cell/packaging cell may also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter). The vectors may be self-inactivating (SIN) vectors in which the viral enhancer and promoter sequences have been deleted. SIN vectors can be generated and transduce non-dividing cells *in vivo* with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR. Vectors may be integration-defective. Integration defective lentiviral vectors (IDLVs) can be produced, for example, either by packaging the vector with catalytically inactive integrase (such as an HIV integrase bearing the D64V mutation in the catalytic site; Naldini et al. (1996) Science, 272(5259):263-277, doi: https://doi.org/10.1126/ science.272.5259.263; Naldini et al. (1996) PNAS, 93(21):11382-11388, PMID: 8876144; and Leavitt et al. (1996) J. Virol., 70(2):721-728, doi: https://doi.org/10.1128/ivi.70.2.721-728.1996) or by modifying or deleting essential *att* sequences from the vector LTR (Nightingale et al. (2006) Mol. Ther., 13(6):1121-1132, doi: https://doi.org/10.1016/i.vmthe.2006.01.008), or by a combination of the above.

The vector of the present invention may be in the form of a viral vector particle. The term "lentivirus vector particle" used herein refers to a complete virion, comprising: (i) the genetic material of the virus, which includes a nucleic acid sequence to be transduced and optionally encoding a gene such as a transgene as described herein; (ii) a protein coat (the capsid) which surrounds the genetic material; and optionally (iii) a lipid envelope. Thus such references herein include the nucleic acid comprised in the virion/virus particle. The terms "virus particle", "virus vector particle" (e.g. "lentivirus vector particle") and "virion" may be used interchangeably herein. Preferably, the virus vector particles provided herein comprise an envelope. The envelope is a lipid membrane acquired from the intracellular membrane of the host cell, such as the Golgi membrane or the outer membrane of the cell, which also includes viral glycoproteins (e.g. GP64 as described herein) and optionally other proteins associated with the host cell membrane and/or producer cell membrane (e.g. CD47, or a functional fragment thereof, as described herein). These glycoproteins are the main drivers of human pathogenic virus infectivity, driving fusion of the virus particle with the cell membrane and subsequent viral entry. Thus, in certain embodiments the viral vector is pseudotyped to enter cells via an endocytosis-dependent mechanism.

### GP64 Envelope Protein

Baculovirus (*Autographa californica* nuclear polyhedrosis virus, AcNPV) is an enveloped insect virus, and baculovirus vectors have been shown to efficiently transduce human hepatocytes and explanted human live tissue, as well as other cell types. The major envelope fusion glycoprotein of baculovirus, GP64, is found on the surface of both infected cells and budded virions, where it exists as a homotrimer. It defines the range of cells capable of being infected by the virus and the efficiency by determining the viral receptor preferences to the host cell. Baculoviruses enter host cells by endocytosis followed by a low pH-induced fusion of the viral envelope with the endosomal membrane, allowing entry into the cell's cytoplasm. GP64 is critically involved in this membrane fusion upon virus entry, as well as in the efficient budding of virions from the infected cell. To date, GP64-LVs have been shown to efficiently deliver nucleic acid to various cell types, including hepatic cells and hepatic cell lines (e.g. Huh7 cells), and have tropism restricted against haematopoietic cell lineages (Schauber *et al.* (2004)). In certain embodiments of the present invention are lentiviral vector particles (LVs) pseudotyped with the baculovirus-derived GP64 envelope protein. Thus, in one aspect there is provided a GP64-LV as described herein.

In one embodiment, the GP64 envelope protein comprises the following amino acid sequence or a variant or functional fragment thereof:

MVSAIVLYVLLAAAAHSAFAAEHCNAQMKTGPYKIKNLDITPPKETLQKDVEITIVETDYNENV
IIGYKGYYQAYAYNGGSLDPNTRVEETMKTLNVGKEDLLMWSIRQQCEVGEELIDRWGSDS
DDCFRDNEGRGQWVKGKELVKRQNNNHFAHHTCNKSWRCGISTSKMYSRLECQDDTDE
CQVYILDAEGNPINVTVDTVLHRDGVSMILKQKSTFTTRQIKAACLLIKDDKNNPESVTREHC
LIDNDIYDLSKNTWNCKFNRCIKRKVEHRVKKRPPTWRHNVRAKYTEGDTATKGDLMHIQE
ELMYENDLLKMNIELMHAHINKLNNMLHDLIVSVAKVDERLIGNLMNNSVSSTFLSDDTFLLM
PCTNPPAHTSNCYNNSIYKEGRWVANTDSSQCIDFSNYKELAIDDDVEFWIPTIGNTTYHDS
WKDASGWSFIAQQKSNLITTMENTKFGGVGTSLSDITSMAEGELAAKLTSFMFGHVVNFVII
LIVILFLYCMIRNRNRQY (SEQ ID NO: 1).

Thus, according to this embodiment the GP64 envelope protein comprises an amino acid sequence of SEQ ID NO: 1, or a variant or functional fragment thereof. Such variants and functional fragments (including functional fragments of said variants but more suitably functional fragments of non-variants) include those which promote the binding and fusion of the viral vector to the cell membrane in a similar manner or to a similar extent to full length GP64. For example, a variant or functional fragment of GP64, or a functional fragment of a variant of GP64, when used to pseudotype a lentiviral vector particle will provide efficient transduction of nucleic acids to hepatic cells, in particular liver endothelial cells (preferably LSECs) *in vivo,* and have restricted tropism against haematopoietic cell lineages. Thus, in one embodiment a variant or functional fragment of GP64, or a functional fragment of a variant of GP64, promotes viral entry into a target cell. In a particular embodiment, the functional fragment of GP64 promotes viral entry. In further embodiments, the variant (or functional fragment thereof) comprises about 60%, about 70%, about 80%, about 90% or about 100% sequence identity to the amino acid sequence of SEQ ID NO: 1. In a further embodiment, the variant of GP64 (or a functional fragment thereof) comprises about 307, about 308, about 358, about 359, about 409, about 410, about 460 or about 461 amino acids of SEQ ID NO: 1. In particular, the variant (or functional fragment thereof) comprises about 80%, about 85%, about 90%, about 95% or about 100% sequence identity to SEQ ID NO: 1, preferably 91%, 92%, 93%, 94%, 95%, 96%, 97%, 98% or 99% sequence identity to SEQ ID NO: 1. In a yet further embodiment, the variant (or functional fragment thereof) comprises about 435, about 436, about 486 or about 487 amino acids of SEQ ID NO: 1, preferably about 465, about 466, about 471, about 472, about 476, about 477, about 481, about 482, about 486, about 487, about 491, about 492, about 496, about 497, about 501, about 502, about 506 or about 507 amino acids of SEQ ID NO: 1. In another embodiment, the GP64 envelope protein consists of an amino acid sequence of SEQ ID NO: 1.

In a further embodiment, the GP64 envelope protein comprises an amino acid sequence encoded by the following nucleotide sequence:

ATGGTAAGCGCTATTGTTTTATATGTGCTTTTGGCGGCGGCGCATTCTGCCTTTGCG
GCGGAGCACTGCAACGCGCAAATGAAGACGGGTCCGTACAAGATTAAAAACTTGGACAT
TACCCCGCCCAAGGAAACGCTGCAAAAGGACGTGGAAATCACCATCGTGGAGACGGAC
TACAACGAAAACGTGATTATCGGCTACAAGGGGTACTACCAGGCGTATGCGTACAACGG
CGGCTCGCTGGATCCCAACACACGCGTCGAAGAAACCATGAAAACGCTGAATGTGGGC
AAAGAGGATTTGCTTATGTGGAGCATCAGGCAGCAGTGCGAGGTGGGCGAAGAGCTGA
TCGACCGTTGGGGCAGTGACAGCGACGACTGTTTTCGCGACAACGAGGGCCGCGGCC
AGTGGGTCAAAGGCAAAGAGTTGGTGAAGCGGCAGAATAACAATCACTTTGCGCACCA
CACGTGCAACAAATCGTGGCGATGCGGCATTTCCACTTCGAAAATGTACAGCAGGCTCG
AGTGCCAGGACGACACGGACGAGTGCCAGGTATACATTTTGGACGCTGAGGGCAACCC
CATCAACGTGACCGTGGACACTGTGCTTCATCGAGACGGCGTGAGTATGATTCTCAAAC
AAAAGTCTACGTTCACCACGCGCCAAATAAAAGCTGCGTGTCTGCTCATTAAAGATGACA
AAAATAACCCCGAGTCGGTGACACGCGAACACTGTTTGATTGACAATGATATATATGATCT
TTCTAAAAACACGTGGAACTGCAAGTTTAACAGATGCATTAAACGCAAAGTCGAGCACCG
AGTCAAGAAGCGGCCGCCCACTTGGCGCCACAACGTTAGAGCCAAGTACACAGAGGGA
GACACTGCCACCAAAGGCGACCTGATGCATATTCAAGAGGAGCTGATGTACGAAAACGA
TTTGCTGAAAATGAACATTGAGCTGATGCATGCGCACATCAACAAGCTAAACAATATGCT
GCACGACCTGATAGTCTCCGTGGCCAAGGTGGACGAGCGTTTGATTGGCAATCTCATGA
ACAACTCTGTTTCTTCAACATTTTTGTCGGACGACACGTTTTTGCTGATGCCGTGCACCA
ATCCGCCGGCACACACCAGTAATTGCTACAACAACAGCATCTACAAAGAAGGGCGTTGG
GTGGCCAACACGGACTCGTCGCAATGCATAGATTTTAGCAACTACAAGGAACTAGCAATT
GACGACGACGTCGAGTTTTGGATCCCGACCATCGGCAACACGACCTATCACGACAGTT
GGAAAGATGCCAGCGGCTGGTCGTTTATTGCCCAACAAAAAAGCAACCTCATAACCACC
ATGGAGAACACCAAGTTTGGCGGCGTCGGCACCAGTCTGAGCGACATCACTTCCATGG
CTGAAGGCGAATTGGCCGCTAAATTGACTTCGTTCATGTTTGGTCATGTAGTTAACTTTG
TAATTATATTAATTGTGATTTTATTTTTGTACTGTATGATTAGAAACCGTAATAGACAATATTA
A (SEQ ID NO: 2).

In a yet further embodiment, the GP64 envelope protein comprises a variant or functional fragment of the amino acid sequence encoded by SEQ ID NO: 2 (including functional fragments of said variants but more suitably functional fragments of non-variants), for example wherein the GP64 envelope protein comprises a functional amino acid sequence encoded by a fragment of SEQ ID NO: 2. Such variants or functional fragments thereof may comprise any hereinbefore mentioned precent sequence identity to the amino acid sequence encoded by SEQ ID NO: 2. In a particular embodiment, the GP64 envelope protein comprises a functional fragment of the amino acid sequence encoded by SEQ ID NO: 2. In another embodiment, the GP64 envelope protein consists of an amino acid sequence encoded by SEQ ID NO: 2.

Provided herein are methods and pseudotyped LVs for the transduction of liver endothelial cells. As demonstrated herein, the methods and LVs of the present invention provide efficient and specific transduction of liver endothelial cells over non-liver endothelium. As will be appreciated by the skilled person, the majority of endothelial cells in the liver are liver sinusoidal endothelial cells (LSECs). Thus, in particularly preferred embodiments, the liver endothelial cells are liver sinusoidal endothelial cells (LSECs). LSECs are cells found in the liver of mammals which form the lining of small blood vessels in the liver, also known as hepatic sinusoids. They make up a small total of liver volume but provide a very large surface area for contacting blood. While considered endothelial cells, the structure of LSECs differs from other endothelia found in other organs in that they contain open pores or fenestrae over approximately 20% of their surface in 'sieve plate' groups (Cogger & Couteur (2009) The Liver, doi: https://doi.orq/10.1002/ 9780470747919.ch27). These pores/fenestrae filter fluid between the sinusoidal lumen and the space of Disse, and are crucial for lipoprotein traffic between the hepatocytes and the sinusoidal lumen, clearing blood-borne waste (Fraser et al. (2012) Pathology, 44(3):181-186, doi: https://doi.ora/10.1097/ PAT.0b013e328351bcc8). This unique structure of LSECs compared to other endothelial cells may, in part, explain their ability to be preferentially transduced using GP64-LV as described herein. LSECs are also the physiological source of coagulation factor VIII (Follenzi *et al.* (2008); and Zanolini *et al.* (2015)).

Thus, in particular embodiments of the present invention the methods and GP64 pseudotyped lentiviral vector particles provided herein transduce liver sinusoidal endothelial cells (LSECs). In further embodiments, the methods and GP64-LVs display improved tropism for liver endothelial cells, in particular LSECs, compared to non-GP64 pseudotyped LVs, such as LVs pseudotyped with VSV.G (VSV.G-LV). In one embodiment, the methods and GP64-LVs have a higher efficiency of transduction of liver endothelial cells, in particular LSECs, compared to VSV.G-LV, such as when measured by the number/percent transduced cells (e.g. GFP+ cells when the nucleic acid delivered by the LV encodes for GFP) and/or by the amount of gene product produced by the cell (e.g. released by the cell when the nucleic acid delivered by the LV encodes for a is secreted/released product, such as factor VIII). In a further embodiment, the GP64-LV provided herein (including CD47hi GP64-LVs) transduce liver endothelial cells, in particular LSECs, at a higher efficiency when used at a low multiplicity of infection (MOI) to the number of liver endothelial cells/LSECs compared to VSV.G-LV.

As demonstrated herein, the methods and GP64-LVs of the present invention preferentially transduce liver endothelial cells compared to endothelial cells of other tissues and organs. In particular, it is demonstrated that the methods and GP64-LVs of the invention preferentially transduce LSECs with reduced tropism for/restricted tropism against plasmacytoid dendritic cells (pDCs). Thus, in one embodiment the methods and GP64-LVs provided herein have reduced tropism for non-liver endothelial cells. In another embodiment, the methods and GP64-LVs provided herein have reduced tropism for haematopoietic cells. In a further embodiment, tropism is restricted against haematopoietic cells. In a yet further embodiment, tropism is reduced for antigen presenting cells (APCs), and/or tropism is restricted against APCs. Such APCs include, without limitation the classical or 'professional' APCs: dendritic cells, macrophages, Langerhans cells and B cells, which express MHC class II molecules along with co-stimulatory molecules and pattern recognition receptors, as well as non-classical APCs. In one embodiment, tropism is reduced for pDCs and/or tropism is restricted against pDCs. In a further embodiment, tropism is reduced for lymphoid tissues or organs (e.g. the spleen) and/or tropism is restricted against lymphoid tissues or organs. In a yet further embodiment, tropism is reduced for cells of the spleen and/or tropism is restricted against cells of the spleen. As described hereinbefore, reduced tropism for or restricted tropism against haematopoietic cells provides the advantage of reduced off-target effects, including the reduced likelihood of an immune response being elicited against the transduced gene product. Immune responses to the transduced gene product reduce or completely abrogate the therapeutic effect provided by the transduced gene product, as seen in instances where VSV.G-LVs are used (Dyall *et al.* (2007); Chinnasamy *et al.* (2000); Metharom *et al.* (2001); and VandenDriessche *et al.* (2002)). Furthermore, reduced tropism for non-liver endothelial cells provides the advantage of increasing the number of lentiviral vector particles available to transduce the target liver endothelial cells by reducing the number of lentiviral vector particles which may be 'soaked up' by these non-target cells. This effectively increases the multiplicity of infection (MOI) relative to the target liver endothelial cells to be transduced.

Also as shown by the data presented herein, the methods and GP64-LVs of the invention preferentially transduce LSECs over hepatocytes, Kupffer cells (KCs) and liver macrophages. Thus, in another embodiment the methods and GP64-LVs provided herein have reduced tropism for other cells of the liver than LSECs. In one embodiment, tropism is reduced for hepatocytes and/or tropism is restricted against hepatocytes. In a further embodiment, tropism is reduced for KCs and/or tropism is restricted against KCs. In a yet further embodiment, tropism is reduced for liver macrophages and/or tropism is restricted against liver macrophages. The hereinbefore mentioned advantages of increasing the number of lentiviral vector particles available to transduce the target liver endothelial cells will be readily appreciated to also apply to these embodiments.

### Lentiviral Vector Particles Comprising High Levels of CD47, or Functional Fragments Thereof

In further embodiments, the methods and GP64-LVs provided herein comprise high levels of the natural phagocytosis inhibitor CD47, or a variant or functional fragment thereof. In particular embodiments, the GP64-LVs comprise high levels of CD47, or a variant or functional fragment thereof, on their surface. In further embodiments, the lentiviral vector particle comprises high levels of CD47, or a variant or functional fragment thereof, on its surface. References herein to LVs with "high levels of CD47" or "CD47hi" LVs may be used interchangeably herein and refer to lentiviral vector particles with increased levels of CD47, or a variant or functional fragment thereof, which may be obtained from host/producer cells that are modified to express increased amounts of CD47, or a variant or functional fragment thereof, on its surface compared to unmodified host/producer cells. Methods of increasing the expression of CD47, or a variant or functional fragment thereof, in host/producer cells are known in the art and include, without limitation transfection or viral transduction of an expression cassette comprising a coding sequence of the CD47, variant or functional fragment. The host/producer cell may have at least about 2-fold, at least about 3-fold, at least about 4-fold, at least about 5-fold, at least about 10-fold, at least about 20-fold or at least about 30-fold more CD47 (or variant or functional fragment thereof) on its cell surface than an unmodified cell. In particular, the host/producer cell has from about 5-fold to about 30-fold more CD47 (or a variant or functional fragment thereof) on its surface than an unmodified cell.

The amount of CD47 (or variant or functional fragment thereof) on the surface of the lentiviral vector particle may be enough to reduce uptake by professional phagocytes. Any suitable assay to quantify the amount of CD47 polypeptides (or variants or functional fragments thereof) present on the surface of the lentiviral vector particle may be used, for example by immunostaining for CD47 (or a variant or functional fragment thereof) followed by total internal reflection fluorescence microscopy (e.g. as described in US2010/0316570, the assays of which for determining CD47 density/presence are hereby specifically incorporated by reference in their entirety), or by immunostaining for CD47 (or a variant or functional fragment thereof) followed by electron microscopy (e.g. as described in Milani et al. ((2019) Sci. Transl. Med., 11(493):eaav7325, doi: https://doi.org/10.1126/ scitranslmed.aav7325, the assays of which for determining CD47 density/presence are hereby specifically incorporated by reference in their entirety). The CD47 (or variants or functional fragments thereof) may be present in a density of at least about 20 molecules/µm², at least about 25 molecules/µm², at least about 30 molecules/µm², at least about 35 molecules/µm², at least about 40 molecules/µm², at least about 45 molecules/µm², at least about 50 molecules/µm², at least about 60 molecules/µm², at least about 70 molecules/µm², at least about 80 molecules/µm², at least about 90 molecules/µm², at least about 100 molecules/µm², at least about 150 molecules/µm², at least about 200 molecules/µm², at least about 250 molecules/µm², at least about 300 molecules/µm², at least about 350 molecules/µm², at least about 400 molecules/µm², at least about 450 molecules/µm², at least about 500 molecules/µm², at least about 600 molecules/µm², at least about 700 molecules/µm², at least about 800 molecules/µm², at least about 900 molecules/µm² or at least about 1000 molecules/µm². The CD47 (or variants or functional fragments thereof) may be present in a density of about 1000 molecules/µm² or less, about 500 molecules/µm² or less or about 250 molecules/µm² or less. The CD47 (or variants or functional fragments thereof) may be present in a density of from about 20 molecules/µm² to about 1000 molecules/µm², from about 20 molecules/µm² to about 500 molecules/µm² or from about 20 molecules/µm² to about 250 molecules/µm². Wherein electron microscopy is used, the CD47 (or variants or functional fragments thereof) may be detected in an amount of at least about 10 gold particles/lentiviral particle, at least about 15 gold particles/lentiviral particle or at least about 20 gold particles/lentiviral particle. The CD47 (or variants or functional fragments thereof) may be detected in an amount of about 100 gold particles/lentiviral particle or less, about 80 gold particles/lentiviral particle or less or about 60 gold particles/lentiviral particle or less. The CD47 (or variants or functional fragments thereof) may be detected in an amount of from about 10 to about 100 gold particles/lentiviral particle, from about 15 to about 80 gold particles/lentiviral particle or from about 20 to about 60 gold particles/lentiviral particle.

CD47, also known as integrin associated protein (IAP), is a transmembrane protein that in humans is encoded by the CD47 gene and the human polypeptide sequence is known and recorded as UniProt accession number Q08722. Phagocytosis is physiologically inhibited by CD47, which is a ubiquitously expressed ligand of the signal regulatory protein α (SIRP-α) receptor, that is expressed by professional phagocytes. CD47, expressed on the surface of host/producer cells may be incorporated into lentiviral vectors when they bud from said host/producer cells. It has been previously shown that LVs with high surface content CD47 (CD47hi-LV) are more resistant to capture by professional phagocytes. This is supported by the data presented herein which demonstrate higher transduction (as shown by vector copy number; VCN herein) in LSECs when CD47hi GP64-LVs are used. By contrast, no increase in hepatocyte transduction was seen, confirming a high efficiency of gene transfer to LSEC *in vivo* mediated by GP64-LV systemic administration which is further enhanced by combining CD47hi and GP64-pseudotyped LV. Thus, in a further embodiment the CD47hi GP64-LVs provided herein transduce LSECs. In a yet further embodiment, the CD47hi GP64-LVs display improved tropism for LSECs compared to non-GP64 pseudotyped LVs, such as LVs pseudotyped with VSV.G (VSV.G-LV), including CD47hi VSV.G-LV. In a still further embodiment, the CD47hi GP64-LVs have reduced tropism for other cells of the liver than LSECs. In one embodiment, tropism is reduced for hepatocytes and/or tropism is restricted against hepatocytes. In a further embodiment, tropism is reduced for KCs and/or tropism is restricted against KCs. In one embodiment, tropism is reduced for liver macrophages and/or tropism is restricted against liver macrophages. In a yet further embodiment, the CD47hi GP64-LVs have reduced tropism for haematopoietic cells and/or tropism is restricted against haematopoietic cells, such as APCs as described hereinbefore, including pDCs.

Thus, in some embodiments the present methods and GP64-LVs specifically transduce LSECs. In other embodiments, the CD47hi GP64-LVs provided herein specifically transduce LSECs.

"CD47hi" as used herein will be readily appreciated to refer to the complete/full length CD47 protein, as well as variants and functional fragments thereof as described herein. Such variants and functional fragments (including functional fragments of said variants but more suitably functional fragments of non-variants) have been previously described in Fenalti et al. ((2021) Nat. Comms., 12(1):1-14)) and include those which retain the phagocytosis inhibitor function of CD47 and/or which inhibit or reduce phagocytosis compared to a lentivirus vector particle not comprising high levels of CD47 or a fragment thereof on its surface. For example, a variant or functional fragment of CD47 may comprise the extracellular domain which is present on the outside of the virus particle following budding from the producer/host cell, i.e. the variant/fragment may comprise the part of CD47 'seen' by macrophages *in vivo* and which binds to SIRP-α to inhibit phagocytosis. Such extracellular domain variants or fragments of CD47 may be joined to any suitable group/protein/amino acid sequence to anchor into the envelope of the virus particle, e.g. the transmembrane domain of CD47, a lipid head group or any other protein comprised in the virus envelope.

In one embodiment, the CD47 protein comprises the following amino acid sequence or a functional fragment thereof:

MWPLVAALLLGSACCGSAQLLFNKTKSVEFTFCNDTVVIPCFVTNMEAQNTTEVYVKWKFK
GRDIYTFDGALNKSTVPTDFSSAKIEVSQLLKGDASLKMDKSDAVSHTGNYTCEVTELTREG
ETIIELKYRVVSWFSPNENILIVIFPIFAILLFWGQFGIKTLKYRSGGMDEKTIALLVAGLVITVIVI
VGAILFVPGEYSLKNATGLGLIVTSTGILILLHYYVFSTAIGLTSFVIAILVIQVIAYILAVVGLSLCI

AACIPMHGPLLISGLSILALAQLLGLVYMKFVASNQKTIQPPRKAVEEPLNAFKESKGMMNDE
(SEQ ID NO: 3).

In another embodiment, the CD47 variant or functional fragment comprises the extracellular domain of CD47. In a further embodiment, the CD47 variant or functional fragment thus comprises the following amino acid sequence:

LLFNKTKSVEFTFCNDTVVIPCFVTNMEAQNTTEVYVKWKFKGRDIYTFDGALNKSTVPTDF
SSAKIEVSQLLKGDASLKMDKSDAVSHTGNYTCEVTELTREGETIIELKYRV (SEQ ID NO: 4).

Thus, according to these embodiments the CD47 protein comprises an amino acid sequence of SEQ ID NO: 3, or a variant or functional fragment thereof. Such variants and functional fragments (including functional fragments of said variants but more suitably functional fragments of non-variants) include those described hereinbefore, in particular the extracellular domain of CD47. Thus, in a further embodiment the CD47 variant or functional fragment comprises an amino acid sequence of SEQ ID NO: 4. In another embodiment, the variant of CD47 (or a functional fragment thereof) comprises about 60%, about 70%, about 80%, about 90% or about 100% sequence identity to the amino acid sequence of SEQ ID NO: 3 and/or SEQ ID NO: 4. In a further embodiment, the variant of CD47 (or a functional fragment thereof) comprises about 193, about 194, about 226, about 227, about 258, about 259, about 290 or about 291 amino acids of SEQ ID NO: 3 and/or about 68, about 69, about 79, about 80, about 91, about 92, about 102 or about 103 amino acids of SEQ ID NO: 4. In particular, the variant (or functional fragment thereof) comprises about 80%, about 85%, about 90%, about 95% or about 100% sequence identity to SEQ ID NO: 3 and/or SEQ ID NO: 4, preferably 91%, 92%, 93%, 94%, 95%, 96%, 97%, 98% or 99% sequence identity to SEQ ID NO: 3 and/or SEQ ID NO: 4. In a yet further embodiment, the variant (or functional fragment thereof) comprises about 274, about 275, about 306 or about 307 amino acids of SEQ ID NO: 3 and/or about 96, about 97, about 108 or about 109 amino acids of SEQ ID NO: 4, preferably about 293, about 294, about 297, about 298, about 300, about 301, about 303, about 304, about 306, about 307, about 310, about 311, about 313, about 314, about 316, about 317, about 319 or about 320 amino acids of SEQ ID NO: 3 and/or about 103, about 104, about 105, about 106, about 107, about 108, about 109, about 110, about 111, about 112 or about 113 amino acids of SEQ ID NO: 4. In another embodiment, the CD47 protein consists of an amino acid sequence of SEQ ID NO: 3.

In a further embodiment, the CD47 protein comprises an amino acid sequence encoded by the following nucleotide sequence:

AATTCGGATCCGCCACCATGTGGCCTCTCGTGGCCGCTCTGCTGCTCGGGAGCGCTTG
TTGCGGCAGCGCCCAGCTGCTGTTCAACAAAACCAAGTCCGTCGAGTTCACCTTCTGC
AACGACACAGTGGTGATCCCCTGCTTCGTCACCAACATGGAGGCTCAGAATACCACCGA
GGTCTACGTCAAGTGGAAATTCAAGGGCAGAGACATCTACACCTTCGACGGAGCCCTCA
ACAAGAGCACAGTGCCTACCGACTTTTCCAGCGCCAAGATTGAGGTGAGCCAACTCCT
GAAGGGAGACGCCAGCCTGAAGATGGACAAGAGCGATGCCGTCAGCCACACAGGAAA
CTACACCTGCGAGGTGACAGAGCTCACCAGAGAGGGCGAGACCATCATCGAGCTCAAA
TACAGAGTGGTGTCCTGGTTCTCCCCCAACGAGAACATCCTCATCGTGATCTTCCCCAT
CTTCGCCATCCTGCTGTTCTGGGGCCAGTTCGGCATCAAAACCCTGAAGTATAGATCCG
GCGGCATGGACGAGAAAACAATCGCCCTGCTGGTGGCCGGCCTCGTGATTACCGTGAT
CGTCATCGTGGGCGCCATCCTCTTCGTGCCCGGAGAGTACAGCCTCAAGAACGCCACC
GGCCTGGGCCTGATTGTGACCTCCACAGGCATTCTGATCCTGCTGCACTACTACGTGTT
CAGCACAGCCATTGGCCTCACAAGCTTCGTGATCGCCATCCTGGTCATCCAGGTGATCG
CCTACATCCTCGCCGTGGTCGGACTCAGCCTCTGTATTGCCGCTTGCATCCCCATGCAC
GGACCCCTCCTGATCTCCGGCCTCAGCATTCTGGCTCTCGCTCAGCTGCTCGGCCTGG
TGTACATGAAGTTCGTCGCCAGCAACCAGAAGACCATCCAACCCCCCAGAAAGGCCGT
CGAAGAGCCTCTGAACGCCTTTAAGGAGAGCAAGGGCATGATGAACGACGAGTGAGCG
GCCGCG (SEQ ID NO: 5).

In another embodiment, the CD47 protein consists of an amino acid sequence encoded by SEQ ID NO: 5.

In a yet further embodiment, the CD47 protein comprises a variant or functional fragment of the amino acid sequence encoded by SEQ ID NO: 5 (including functional fragments of said variants but more suitably functional fragments of non-variants), for example wherein the CD47 protein comprises a functional amino acid sequence encoded by a fragment of SEQ ID NO: 5. Such variants or functional fragments thereof may comprise any hereinbefore mentioned precent sequence identity to the amino acid sequence encoded by SEQ ID NO: 5. In a particular embodiment, the CD47 protein comprises a functional fragment of the amino acid sequence encoded by SEQ ID NO: 5.

Thus, in one embodiment, the CD47 functional fragment comprises an amino acid sequence encoded by the following nucleotide sequence:

CTGCTGTTCAACAAAACCAAGTCCGTCGAGTTCACCTTCTGCAACGACACAGTGGTGAT
CCCCTGCTTCGTCACCAACATGGAGGCTCAGAATACCACCGAGGTCTACGTCAAGTGG

AAATTCAAGGGCAGAGACATCTACACCTTCGACGGAGCCCTCAACAAGAGCACAGTGC
CTACCGACTTTTCCAGCGCCAAGATTGAGGTGAGCCAACTCCTGAAGGGAGACGCCAG
CCTGAAGATGGACAAGAGCGATGCCGTCAGCCACACAGGAAACTACACCTGCGAGGTG
ACAGAGCTCACCAGAGAGGGCGAGACCATCATCGAGCTCAAATACAGAGTG (SEQ ID
NO: 6).

The amino acid sequence encoded by SEQ ID NO: 6 corresponds to the extracellular domain of CD47. Thus, according to this embodiment the CD47 variant or functional fragment, in particular the functional fragment, is the extracellular domain of CD47 (e.g. is the amino acid of SEQ ID NO: 4).

### Lentiviral Vector Particles Comprising Low Levels of MHC-I or MHC-I Free Lentiviral Vector Particles

The lentiviral vector particles described herein may comprise low levels of MHC-I on their surface or may be MHC-I "free". In certain embodiments, the lentivirus vector particle is MHC-I free. References herein to "low levels" of MHC-I or "MHC-I free" refer to lentivirus vector particles which are substantially devoid/free of one or more MHC-I molecules on its surface which may be obtained from host/producer cells that are modified to have reduced amounts of MHC-I on their surface compared to unmodified host/producer cells. The major histocompatibility complex class I (MHC-I) is a heterodimeric membrane protein that is displayed on the outer leaflet of the cell membrane (see e.g. Penn & Ilmonen (2005) Major Histocompatibility Complex (MHC), in eLS (Ed.), doi: https://doi.org/10.1038/ npg.els.0003986). MHC-I functions to bind and display peptide fragments of proteins to the extracellular environment where they may be recognised by CD8+ cytotoxic T cells. Peptide fragments generated from normal cellular proteins will not activate cytotoxic T cells due to central and peripheral tolerance mechanisms. However, foreign peptides (e.g. those originating from viral proteins) will cause activation of an immune response to destroy the cell. An allogeneic MHC-I protein itself may be recognised by the immune system. For example, antibodies may bind MHC-I epitopes directly. As a result, lentiviral vectors that comprise MHC-I molecules originating from an allogeneic source may be targeted and neutralised by the immune system.

Any suitable assay to quantify the amount of MHC-I molecules present on the surface of the lentiviral vector particle may be used, including without limitation by immunostaining for MHC-I followed by electron microscopy (e.g as described in Milani et al. (2017) EMBO Mol. Med., 9(11):1558-1573, doi: https://doi.org/10.15252/emmm.201708148; the assays of which for determining MHC-I density/presence are hereby specifically incorporated by reference in their entirety). The lentiviral vector of the present invention may be obtained from a host/producer cell with low levels of MHC-I on its surface (an MHC-I low host/producer cell) or an MHC-I free host/producer cell. An "MHC-I low host/producer cell" may refer to a host/producer cell with reduced levels of one or more MHC-I molecule on its surface. An "MHC-I free host/producer cell" may refer to a host cell which is substantially devoid of or free of one or more MHC-I molecule on its surface. An MHC-I low or MHC-I free host/producer cell may be genetically engineered to decrease expression of MHC-I on the cell surface. For example, the cell may comprise a genetically engineered disruption of a gene encoding β2-microglobulin and/or a disruption of a gene encoding an MHC-I α chain. β2-microglobulin stabilises MHC-I, thus cells deficient in β2-microglobulin will exhibit decreased expression of MHC-I on the surface of the cell. Methods for genetic engineering to decrease protein expression are known in the art.

In further embodiments, the lentivirus vector particle comprises high levels of CD47 and low levels of MHC-I on its surface. In a yet further embodiment, the lentivirus vector particle is CD47hi and MHC-I free. In a still further embodiment, the lentivirus vector particle is CD47hi and comprises low levels of MHC-I on its surface. Thus, in some embodiments the lentivirus vector particle may be obtained from a CD47hi/MHC-I free host/producer cell or a CD47hi/MHC-I low host/producer cell.

### Liver Sinusoidal Endothelial Cells (LSECs)

The involvement of LSECs has been implicated in several liver diseases, including without limitation liver fibrosis, atherosclerosis and autoimmunity. Decreased LSEC fenestration is seen in liver fibrosis, and an organised basal lamina develops in the space of Disse, a process called capillarisation, which precedes the onset of liver fibrosis (DeLeve (2015) Hepatology, 61(5):1740-1746, doi: https://doi.org/10.1002%2Fhep.27376). Normally differentiated LSECs prevent hepatic stellate cell activation and promote reversion to quiescence, whereas capillarised LSECs do not. In atherosclerosis, reduced porosity of LSECs (e.g. due to liver cirrhosis, diabetes mellitus or old age) may lead to prolonged postprandial lipoproteinemia and increased circulatory cholesterol levels, with increased risk for development of atherosclerosis. In support of the role of LSECs in autoimmunity, Small soluble IgG-antigen immune complexes have been shown to be cleared in the mouse mainly via the LSEC FcyRllb2, and deletion of this receptor causes spontaneous autoimmunity and SLE-like disease in mice. Additionally, the scavenging of blood borne DNAs is chiefly by scavenger receptor-mediated uptake in LSECs (SLE is associated with the generation of systemic anti-DNA antibodies). Furthermore, as discussed in Gracia-Sancho et al. ((2021) Nat. Rev. Gastroenterol. Hepatol., 18(6):411-431, doi: https://doi.org/10.1038/s41575-020-00411-3) LSECs can modify their phenotype and negatively affect neighbouring cells and live disease pathophysiology in, e.g. a background of chronic liver injury. Thus, LSEC functions and dysregulation has been reported in a wide number of diseases, including without limitation acute injury (ischaemia-reperfusion injury, drug-induced liver injury and bacterial and viral infection), chronic liver disease (metabolism-associated liver disease, alcoholic steatohepatitis and chronic hepatotoxic injury), hepatic sinusoidal obstruction syndrome and hepatocellular carcinoma. As such, LSECs may the used as therapeutic targets in any one of these diseases, in particular in the treatment of chronic liver disease, cirrhosis of the liver, acute-on-chronic liver failure, liver cancer and hepatocellular carcinoma.

Thus, the methods and GP64-LVs of the invention find utility in the transduction of liver endothelial cells, in particular LSECs, wherein the transduction is for liver regeneration, or for the treatment of a liver disease or haemophilia. Thus, in certain embodiments the nucleic acid described herein comprises, such as further comprises, a coding sequence for a gene for liver regeneration, or a gene defective or deficient in a liver disease or haemophilia. The haemophilia may be haemophilia A or B, in particular haemophilia A. In a further embodiment, the nucleic acid delivered by the methods and LVs of the invention comprises, such as further comprises, a coding sequence for a gene deficient in haemophilia.

### Haemophilia

Haemophilia A is a disease caused by a genetic deficiency of clotting/coagulation factor VIII (also FVIII herein). It leads to increased bleeding, and it is most often inherited as an X-linked recessive trait thus mostly affects males (although cases can be caused by spontaneous mutations). Haemophilia may be moderate/mild or severe, with more frequent bleeding from relatively minor injuries or even spontaneously in severe cases compared to only after surgery or severe trauma in mild cases. Moderate suffers can range within this spectrum. Bleeding can occur anywhere in or on the body, either from superficial/surface abrasions or more seriously in sites such as joints, muscles, the gastrointestinal tract and the brain. Repeated bleeding into joints for example can cause permanent joint damage resulting in chronic arthritis. Treatment for haemophilia can include regular intravenous (i.v.) administration of recombinant or plasma-derived factor VIII, or very mild cases can be managed with desmopressin, a drug which releases stored factor VIII from blood vessel walls (Franchini & Lippi (2011) Blood Transfus., 9(4):377-382, doi: https://doi.ora/ 10.2450%2F2011.0113-10). However, regular factor VIII infusions can lead to the development of inhibitor antibodies by the subject, which inactivate and thus reduce the efficacy of the administered factor VIII protein. These are produced by the subject because no endogenous factor VIII is produced by virtue of their genetic deficiency, and thus is recognised by the body as foreign. While activated factor VIII may be given to individuals with inhibitor antibodies, this can only be used during haemorrhage. In any event, recombinant or activated factor VIII treatment requires regular or quickly administered infusions which are inconvenient, can be time-critical and carry the risk of infection, e.g. through frequent cannulation or through a semi-permanent intravenous port.

By contrast, gene therapies for haemophilia offer long term treatment and may even cure the disease. They greatly reduce the need for regular injections and infusions of factor VIII protein by virtue of providing the nucleic acid sequence encoding factor VIII to the cells of the liver which then produce and release factor VIII into the subject's bloodstream. In other words, factor VIII gene therapy replaces the deficient endogenous version of the factor VIII gene in subjects suffering from haemophilia. In order to provide correct and therapeutic levels of factor VIII in the blood, it is desirable to provide the factor VIII-encoding sequence to those liver cells which physiologically express and release factor VIII in non-haemophilia subjects, i.e. liver endothelial cells, in particular LSECs. As demonstrated by the data presented herein, the methods and GP64-LVs of the present invention provide this desirable outcome by displaying restricted tropism for liver endothelial cells, in particular for LSECs. Such correct/therapeutic factor VIII levels will therefore lead to treatment of bleeding disorders and the correction of the abnormal bleeding phenotype seen in haemophilia, such as haemophilia A. Thus, in one embodiment the nucleic acid provided by the LVs herein comprises a sequence encoding factor VIII. In a further embodiment, the factor VIII-encoding sequence is a human sequence, such as a human codon-optimised factor VIII transgene (represented herein by SEQ ID NO: 7).

In particular embodiments, the factor VIII may be any of the sequences disclosed in WO 2017/212460, the factor VIII coding sequences of which are specifically incorporated by reference in their entirety. In a further particular embodiment, the factor VIII coding sequence may comprise or consist of the following nucleotide sequence or a variant or functional fragment thereof (including a functional fragment of said variants):

ATGCAGATTGAGCTGTCCACTTGTTTCTTCCTGTGCCTCCTGCGCTTCTGTTTCTCCGCC
ACTCGCCGGTACTACCTTGGAGCCGTGGAGCTTTCATGGGACTACATGCAGAGCGACC
TGGGCGAACTCCCCGTGGATGCCAGATTCCCCCCCCGCGTGCCAAAGTCCTTCCCCTT
TAACACCTCCGTGGTGTACAAGAAAACCCTCTTTGTCGAGTTCACTGACCACCTGTTCA
ACATCGCCAAGCCGCGCCCACCTTGGATGGGCCTCCTGGGACCGACCATTCAAGCTGA
AGTGTACGACACCGTGGTGATCACCCTGAAGAACATGGCGTCCCACCCCGTGTCCCTG
CATGCGGTCGGAGTGTCCTACTGGAAGGCCTCCGAAGGAGCTGAGTACGACGACCAGA
CTAGCCAGCGGGAAAAGGAGGACGATAAAGTGTTCCCGGGCGGCTCGCATACTTACGT

GTGGCAAGTCCTGAAGGAAAACGGACCTATGGCATCCGATCCTCTGTGCCTGACTTACT
CCTACCTTTCCCATGTGGACCTCGTGAAGGACCTGAACAGCGGGCTGATTGGTGCACTT
CTCGTGTGCCGCGAAGGTTCGCTCGCTAAGGAAAAGACCCAGACCCTCCATAAGTTCAT
CCTTTTGTTCGCTGTGTTCGATGAAGGAAAGTCATGGCATTCCGAAACTAAGAACTCGCT
GATGCAGGACCGGGATGCCGCCTCAGCCCGCGCCTGGCCTAAAATGCATACAGTCAAC
GGATACGTGAATCGGTCACTGCCCGGGCTCATCGGTTGTCACAGAAAGTCCGTGTACTG
GCACGTCATCGGCATGGGCACTACGCCTGAAGTGCACTCCATCTTCCTGGAAGGGCAC
ACCTTCCTCGTGCGCAACCACCGCCAGGCCTCTCTGGAAATCTCCCCGATTACCTTTCT
GACCGCCCAGACTCTGCTCATGGACCTGGGGCAGTTCCTTCTCTTCTGCCACATCTCCA
GCCATCAGCACGACGGAATGGAGGCCTACGTGAAGGTGGACTCATGCCCGGAAGAACC
TCAGTTGCGGATGAAGAACAACGAGGAGGCCGAGGACTATGACGACGATTTGACTGAC
TCCGAGATGGACGTCGTGCGGTTCGATGACGACAACAGCCCCAGCTTCATCCAGATTC
GCAGCGTGGCCAAGAAGCACCCCAAAACCTGGGTGCACTACATCGCGGCCGAGGAAG
AAGATTGGGACTACGCCCCGTTGGTGCTGGCACCCGATGACCGGTCGTACAAGTCCCA
GTATCTGAACAATGGTCCGCAGCGGATTGGCAGAAAGTACAAGAAAGTGCGGTTCATGG
CGTACACTGACGAAACGTTTAAGACCCGGGAGGCCATTCAACATGAGAGCGGCATTCTG
GGACCACTGCTGTACGGAGAGGTCGGCGATACCCTGCTCATCATCTTCAAAAACCAGGC
CTCCCGGCCTTACAACATCTACCCTCACGGAATCACCGACGTGCGGCCACTCTACTCGC
GGCGCCTGCCGAAGGGCGTCAAGCACCTGAAAGACTTCCCTATCCTGCCGGGCGAAAT
CTTCAAGTATAAGTGGACCGTCACCGTGGAGGACGGGCCCACCAAGAGCGATCCTAGG
TGTCTGACTCGGTACTACTCCAGCTTCGTGAACATGGAACGGGACCTGGCATCGGGAC
TCATTGGACCGCTGCTGATCTGCTACAAAGAGTCGGTGGATCAACGCGGCAACCAGATC
ATGTCCGACAAGCGCAACGTGATCCTGTTCTCCGTGTTTGATGAAAACAGATCCTGGTA
CCTCACTGAAAACATCCAGAGGTTCCTCCCAAACCCCGCAGGAGTGCAACTGGAGGAC
CCTGAGTTTCAGGCCTCGAATATCATGCACTCGATTAACGGTTACGTGTTCGACTCGCTG
CAGCTGAGCGTGTGCCTCCATGAAGTCGCTTACTGGTACATTCTGTCCATCGGCGCCCA
GACTGACTTCCTGAGCGTGTTCTTTTCCGGTTACACCTTTAAGCACAAGATGGTGTACGA
AGATACCCTGACCCTGTTCCCTTTCTCCGGCGAAACGGTGTTCATGTCGATGGAGAACC
CGGGTCTGTGGATTCTGGGATGCCACAACAGCGACTTTCGGAACCGCGGAATGACTGC
CCTGCTGAAGGTGTCCTCATGCGACAAGAACACCGGAGACTACTACGAGGACTCCTAC
GAGGATATCTCAGCCTACCTCCTGTCCAAGAACAACGCGATCGAGCCGCGCAGCTTCA
GCCAGAACCCGCCTGTGCTGAAGAGGCACCAGCGAGAAATTACCCGGACCACCCTCCA
ATCGGATCAGGAGGAAATCGACTACGACGACACCATCTCGGTGGAAATGAAGAAGGAA
GATTTCGATATCTACGACGAGGACGAAAATCAGTCCCCTCGCTCATTCCAAAAGAAAACT
AGACACTACTTTATCGCCGCGGTGGAAAGACTGTGGGACTATGGAATGTCATCCAGCCC
TCACGTCCTTCGGAACCGGGCCCAGAGCGGATCGGTGCCTCAGTTCAAGAAAGTGGTG
TTCCAGGAGTTCACCGACGGCAGCTTCACCCAGCCGCTGTACCGGGGAGAACTGAAC

GAACACCTGGGCCTGCTCGGTCCCTACATCCGCGCGGAAGTGGAGGATAACATCATGG
TGACCTTCCGTAACCAAGCATCCAGACCTTACTCCTTCTATTCCTCCCTGATCTCATACGA
GGAGGACCAGCGCCAAGGCGCCGAGCCCCGCAAGAACTTCGTCAAGCCCAACGAGAC
TAAGACCTACTTCTGGAAGGTCCAACACCATATGGCCCCGACCAAGGATGAGTTTGACT
GCAAGGCCTGGGCCTACTTCTCCGACGTGGACCTTGAGAAGGATGTCCATTCCGGCCT
GATCGGGCCGCTGCTCGTGTGTCACACCAACACCCTGAACCCAGCGCATGGACGCCA
GGTCACCGTCCAGGAGTTTGCTCTGTTCTTCACCATTTTTGACGAAACTAAGTCCTGGTA
CTTCACCGAGAATATGGAGCGAAACTGTAGAGCGCCCTGCAATATCCAGATGGAAGATC
CGACTTTCAAGGAGAACTATAGATTCCACGCCATCAACGGGTACATCATGGATACTCTGC
CGGGGCTGGTCATGGCCCAGGATCAGAGGATTCGGTGGTACTTGCTGTCAATGGGATC
GAACGAAAACATTCACTCCATTCACTTCTCCGGTCACGTGTTCACTGTGCGCAAGAAGG
AGGAGTACAAGATGGCGCTGTACAATCTGTACCCCGGGGTGTTCGAAACTGTGGAGATG
CTGCCGTCCAAGGCCGGCATCTGGAGAGTGGAGTGCCTGATCGGAGAGCACCTCCAC
GCGGGGATGTCCACCCTCTTCCTGGTGTACTCGAATAAGTGCCAGACCCCGCTGGGCA
TGGCCTCGGGCCACATCAGAGACTTCCAGATCACAGCAAGCGGACAATACGGCCAATG
GGCGCCGAAGCTGGCCCGCTTGCACTACTCCGGATCGATCAACGCATGGTCCACCAAG
GAACCGTTCTCGTGGATTAAGGTGGACCTCCTGGCCCCTATGATTATCCACGGAATTAAG
ACCCAGGGCGCCAGGCAGAAGTTCTCCTCCCTGTACATCTCGCAATTCATCATCATGTA
CAGCCTGGACGGGAAGAAGTGGCAGACTTACAGGGGAAACTCCACCGGCACCCTGAT
GGTCTTTTTCGGCAACGTGGATTCCTCCGGCATTAAGCACAACATCTTCAACCCACCGAT
CATAGCCAGATATATTAGGCTCCACCCCACTCACTACTCAATCCGCTCAACTCTTCGGAT
GGAACTCATGGGGTGCGACCTGAACTCCTGCTCCATGCCGTTGGGGATGGAATCAAAG
GCTATTAGCGACGCCCAGATCACCGCGAGCTCCTACTTCACTAACATGTTCGCCACCTG
GAGCCCCTCCAAGGCCAGGCTGCACTTGCAGGGACGGTCAAATGCCTGGCGGCCGCA
AGTGAACAATCCGAAGGAATGGCTTCAAGTGGATTTCCAAAAGACCATGAAAGTGACCG
GAGTCACCACCCAGGGAGTGAAGTCCCTTCTGACCTCGATGTATGTGAAGGAGTTCCT
GATTAGCAGCAGCCAGGACGGGCACCAGTGGACCCTGTTCTTCCAAAACGGAAAGGTC
AAGGTGTTCCAGGGGAACCAGGACTCGTTCACACCCGTGGTGAACTCCCTGGACCCCC
CACTGCTGACGCGGTACTTGAGGATTCATCCTCAGTCCTGGGTCCATCAGATTGCATTG
CGAATGGAAGTCCTGGGCTGCGAGGCCCAGGACCTGTAC (SEQ ID NO: 7).

Thus, in certain embodiments the methods and GP64-LVs herein provide an exogenous sequence encoding factor VIII to LSECs. In further embodiments, the GP64-LVs (including CD47hi GP64-LVs) comprise an exogenous sequence in the form of a transgene. In one embodiment, the transgene comprises a gene, element or sequence encoding factor VIII, such that factor VIII is expressed from said transgene. Thus, in a further embodiment the nucleic acid, and optionally the transgene, comprises a factor VIII encoding gene. As will be readily appreciated, the exogenous sequence and/or the transgene may comprise a coding sequence for any one or more gene for liver regeneration and/or deficient in liver disease. In some embodiments, the exogenous sequence encodes for a gene for liver regeneration and/or deficient in liver disease. Said gene may be in addition to the gene deficient in haemophilia.

### Tissue/Organ-Specific (e.g. Liver-Specific) Promoters

In yet further embodiments, the nucleic acid comprises a tissue- or organ-specific promoter operably linked to a coding sequence, such as a coding sequence as described hereinbefore. The term "operably linked" as used herein refers to the promoter being in the same sequence and/or nucleic acid molecule as the coding sequence to which it is operably linked, and suitably positioned and orientated therein for transcription of the coding sequence to be influenced/initiated by (e.g. promoted by/driven from) the promoter, i.e. the coding sequence is under the transcriptional initiation regulation of or is in functional combination with the promoter. In a further embodiment, optionally the exogenous sequence in the form of a transgene comprises such a promoter. In one embodiment, the tissue or organ is the liver, such that the promoter is a liver-specific promoter. Thus, in another embodiment the nucleic acid further comprises a liver-specific promoter. In a still further embodiment, the promoter is endothelial cell-specific such that it is an endothelial cell-specific promoter, in particular an LSEC-specific promoter. Such tissue- or organ-specific promoters may also be cell type/lineage-specific, for example they specifically promote expression of the exogenous sequence/transgene in a particular cell type. Specific expression in a cell type/lineage may also be known as 'restricted expression'. As described hereinbefore, the cells to which the GP64-LVs of the invention (including CD47hi GP64-LVs) preferentially transduce are liver endothelial cells, in particular LSECs. Thus, in one embodiment the promoter is an endothelial cell-specific promoter. In a further embodiment, the nucleic acid comprises said endothelial cell-specific promoter operably linked to a coding sequence. In another embodiment, the promoter is an LSEC-specific promoter, such that the nucleic acid comprises said LSEC-specific promoter operably linked to a coding sequence. According to these embodiments, the endothelial cell-specific and/or LSEC-specific promoter does not drive expression in other cells of the liver, in particular it does not drive expression in hepatocytes and/or KCs. Such promoters will also not drive expression in non-liver cells, such as haematopoietic cells as described hereinbefore.

Promoters which restrict expression to cells of the liver or which promote expression in liver cells but not cells of other tissues or organs, in particular specific cell types in the liver (e.g. liver endothelial cells and LSECs), are known in the art. One particular example is the endogenous human factor VIII (F8) promoter which has been shown to drive expression in LSECs but not in other cells of the liver or in haematopoietic cells (Merlin *et al.* (2019)). Thus, in one embodiment the promoter is the endogenous human factor VIII (F8) promoter. In a further embodiment the nucleic acid comprises said F8 promoter operably linked to a coding sequence. In a yet further embodiment, the F8 promoter drives expression of factor VIII from the nucleic acid, in particular from the factor VIII coding sequence provided by the GP64-LV, specifically in LSECs. Said driving of expression is by virtue of the F8 promoter being operably linked to the factor VIII coding sequence. Thus, as will be readily appreciated expression from the nucleic acid (e.g. of the factor VIII transgene) is specifically in LSECs. Various forms of the F8 promoter are known in the art, in particular as disclosed in WO 2017/212460, the promoter sequences of which are specifically incorporated by reference in their entirety. Of further note in WO 2017/212460 are the enhancer sequences comprised in the sequences referred to therein as "LVpF8.3" (which demonstrated that the sequence from about -3625bp to about -4184bp upstream of the endogenous FVIII coding sequence acts as an enhancer of expression preferably in endothelial cells) and "LVpF8.5" (which demonstrated the presence of a longer enhancer sequence from about -3625bp to about -4429bp upstream of the FVIII coding sequence). Thus, in some embodiments the nucleic acid may comprise a variant or functional fragment of a promoter sequence described herein. Such functional fragments (including functional fragments of promoter variants) will provide substantially the same function as the full length promoter sequence, i.e. drive expression in the tissue/organ of interest. In one embodiment, the promoter is the long form endogenous human F8 promoter. In another embodiment, the promoter is the short form endogenous human F8 promoter. In a further embodiment, the promoter is the very short or nano form endogenous human F8 promoter.

In further embodiments, the F8 promoter is selected from or may comprise any one of the following sequences, or a variant or functional fragment thereof:

CTCGAGGAGCTCACCATGGCTACATTCTGATGTAAAGAGATATATCCTATACCTGGGCCA
AATGTAAACAGCCTGGAAAAGTGTTAGGTTAAAAACAAAACAAAATAAATAAATGAATAAAT
GCCAGGTGGTTATGAGTGCTATTGAGAAAAATGAAGCCAAGAGGGATATCAGTGATGCA
GGTGGGGGTAAAGAGCTTACAACATAAATGTGGTGTTCCATATTTAAACCTCATTCAACA
GGGAAGATTGGAGCTGAAATGTGAAGGAGTTGTGGGAGTGGAACTACGTGGAAATCTG
GGGGAAAGGTGTTTTGGGTAAAAGAAATAGCAAGTGTTGAGGTCCAGGGGCATGAGTG
TGCTTGATATTTTAGGGAAGAGTAAGGAGACCAGTATAACCAGAGTGAGATGAGACTACA
GAGGTCAGGAGAAAGGGCATGCAGACCATGTGGGATGCTCTAGGACCTAGGCCATGGT
AAAGATGTAGGGTTTTACCCTGATGGAGGTCAGAAGCCATTGGAGGATTCTGAGAAGAG
GAGTGACAGGACTCGCTTTATAGTTTTAAATTATAACTATAAATTATAGTTTTTAAAACAATA

GTTGCCTAACCTCATGTTATATGTAAAACTACAGTTTTAAAAACTATAAATTCCTCATACTG
GCAGCAGTGTGAGGGGCAAGGGCAAAAGCAGAGAGACTAACAGGTTGCTGGTTACTCT
TGCTAGTGCAAGTGAATTCTAGAATCTTCGACAACATCCAGAACTTCTCTTGCTGCTGCC
ACTCAGGAAGAGGGTTGGAGTAGGCTAGGAATAGGAGCACAAATTAAAGCTCCTGTTCA
CTTTGACTTCTCCATCCCTCTCCTCCTTTCCTTAAAGGTTCTGATTAAAGCAGACTTATGC
CCCTACTGCTCTCAGAAGTGAATGGGTTAAGTTTAGCAGCCTCCCTTTTGCTACTTCAGT
TCTTCCTGTGGCTGCTTCCCACTGATAAAAAGGAAGCAATCCTATCGGTTACTGCTTAGT
GCTGAGCACATCCAGTGGGTAAAGTTCCTTAAAATGCTCTGCAAAGAAATTGGGACTTTT
CATTAAATCAGAAATTTTACTTTTTTCCCCTCCTGGGAGCTAAAGATATTTTAGAGAAGAAT
TAACCTTTTGCTTCTCCAGTTGAACATTTGTAGCAATAAGTC (SEQ ID NO: 8).

GAGCTCACCATGGCTACATTCTGATGTAAAGAGATATATCCTATACCTGGGCCAAATGTAA
ACAGCCTGGAAAAGTGTTAGGTTAAAAACAAAACAAAATAAATAAATGAATAAATGCCAGG
TGGTTATGAGTGCTATTGAGAAAAATGAAGCCAAGAGGGATATCAGTGATGCAGGTGGG
GGTAAAGAGCTTACAACATAAATGTGGTGTTCCATATTTAAACCTCATTCAACAGGGAAGA
TTGGAGCTGAAATGTGAAGGAGTTGTGGGAGTGGAACTACGTGGAAATCTGGGGGAAA
GGTGTTTTGGGTAAAAGAAATAGCAAGTGTTGAGGTCCAGGGGCATGAGTGTGCTTGAT
ATTTTAGGGAAGAGTAAGGAGACCAGTATAACCAGAGTGAGATGAGACTACAGAGGTCA
GGAGAAAGGGCATGCAGACCATGTGGGATGCTCTAGGACCTAGGCCATGGTAAAGATGT
AGGGTTTTACCCTGATGGAGGTCAGAAGCCATTGGAGGATTCTGAGAAGAGGAGTGAC
AGGACTCGCTTTATAGTTTTAAATTATAACTATAAATTATAGTTTTTAAAACAATAGTTGCCTA
ACCTCATGTTATATGTAAAACTACAGTTTTAAAAACTATAAATTCCTCATACTGGCAGCAGT
GTGAGGGGCAAGGGCAAAAGCAGAGAGACTAACAGGTTGCTGGTTACTCTTGCTAGTG
CAAGTGAATTCTAGAATCTTCGACAACATCCAGAACTTCTCTTGCTGCTGCCACTCAGGA
AGAGGGTTGGAGTAGGCTAGGAATAGGAGCACAAATTAAAGCTCCTGTTCACTTTGACT
TCTCCATCCCTCTCCTCCTTTCCTTAAAGGTTCTGATTAAAGCAGACTTATGCCCCTACTG
CTCTCAGAAGTGAATGGGTTAAGTTTAGCAGCCTCCCTTTTGCTACTTCAGTTCTTCCTG
TGGCTGCTTCCCACTGATAAAAAGGAAGCAATCCTATCGGTTACTGCTTAGTGCTGAGCA
CATCCAGTGGGTAAAGTTCCTTAAAATGCTCTGCAAAGAAATTGGGACTTTTCATTAAATC
AGAAATTTTACTTTTTTCCCCTCCTGGGAGCTAAAGATATTTTAGAGAAGAATTAACCTTTT
GCTTCTCCAGTTGAACATTTGTAGCAATAAGTC (SEQ ID NO: 9).

GTTTTTAAAACAATAGTTGCCTAACCTCATGTTATATGTAAAACTACAGTTTTAAAAACTATA
AATTCCTCATACTGGCAGCAGTGTGAGGGGCAAGGGCAAAAGCAGAGAGACTAACAGG
TTGCTGGTTACTCTTGCTAGTGCAAGTGAATTCTAGAATCTTCGACAACATCCAGAACTT
CTCTTGCTGCTGCCACTCAGGAAGAGGGTTGGAGTAGGCTAGGAATAGGAGCACAAATT
AAAGCTCCTGTTCACTTTGACTTCTCCATCCCTCTCCTCCTTTCCTTAAAGGTTCTGATTA

AAGCAGACTTATGCCCCTACTGCTCTCAGAAGTGAATGGGTTAAGTTTAGCAGCCTCCCT
TTTGCTACTTCAGTTCTTCCTGTGGCTGCTTCCCACTGATAAAAAGGAAGCAATCCTATC
GGTTACTGCTTAGTGCTGAGCACATCCAGTGGGTAAAGTTCCTTAAAATGCTCTGCAAAG
AAATTGGGACTTTTCATTAAATCAGAAATTTTACTTTTTTCCCCTCCTGGGAGCTAAAGATA
TTTTAGAGAAGAATTAACCTTTTGCTTCTCCAGTTGAACATTTGTAGCAATAAGTC (SEQ
ID NO: 10).

TGACTTCTCCATCCCTCTCCTCCTTTCCTTAAAGGTTCTGATTAAAGCAGACTTATGCCCC
TACTGCTCTCAGAAGTGAATGGGTTAAGTTTAGCAGCCTCCCTTTTGCTACTTCAGTTCT
TCCTGTGGCTGCTTCCCACTGATAAAAAGGAAGCAATCCTATCGGTTACTGCTTAGTGCT
GAGCACATCCAGTGGGTAAAGTTCCTTAAAATGCTCTGCAAAGAAATTGGGACTTTTCAT
TAAATCAGAAATTTTACTTTTTTCCCCTCCTGGGAGCTAAAGATATTTTAGAGAAGAATTAA
CCTTTTGCTTCTCCAGTTGAACATTTGTAGCAATAAGTC (SEQ ID NO: 11).

TCGCCACCACTTGGCTTCCGGCACGTGGGGCAGATGTTTCCATTCCCACGGCGGCAGC
GGAAGAGGGAGGGCCGGGCGCGCCGCGGCTGCTTGCAGTCTCCGCAAGCGGCTACA
TCACAGAGCTCAGCGTGCGGTGTCACAGGCCCCGCGGTCCCGCCCAACAGATGCACC
GAGATGCGCGTGCGCAGAAAGCGTCCCGGGGGTGAGGCTCCCTCCCTCGCTCTCCCT
CTACTCCCGCCCCACTCTCCCCCACTTTCCCCCCTCCACCCACCGCGGCCGTCGGGG
CTCGCTCGCTCAGTACCTGGAGGCGAGTTCCTGACGCGACTGCGACTCAATCCTCGCC
TGGTGAAGAATATTTTACCTATGACTCACTGAAAATAAAGACGGCTGAGTGACCGTGTTT
GTTCATGTAAACATTGAACAAATATTTATCGGCTTCTGCGATGTGTCCTACTCTTTTAGTG
GAGGAAGACACATTTTATTTATGTATTTAATTTTTCTTTTGAATTTTACATGCGAGTTATACT
TAATAAAACTCACTTCAAAATATACCTTCAACAGAAAATCCAGCAACAGTTTCTATTATGTT
AGTTAAAACAGCCAGTCTTTTCCTTTACTTTTAAAAATTATTCATAAATGTAATTAGTGAATG
ATAATAAACATTGACATCTGATCCACTGCTTTAGGAGTGACACAAATGAAGTTAACTCAGG
CTATTTTCTTTATAATCATTGTGCTATTGTTTTCTTTTTCTTTTCAATTATACTGCTTAATATAG
GATTTTGTGGCACCATAGGAGTTGAGTAAAAATAAAAGGAATAAAAATATACCTTATCTGG
CCGGGCGCGGTGGCTCACGCCTGTAATTTCAGCAGTTTCGGAGGCCGAGGCGGGCGG
ATCACGCGGTCAGGAGATCGAGGCCATCCTGGCTAACATGGTGAAACCCCGTCTCTACT
AAAAATACAAAAAATTAGCCGGGCATGGTGGCGGCCGCCTGTAGTCCCAGCTACTCGGG
AGGCTGAGGCAGGAGAATGGCGTGAACCCGGGAGGCGGAGCTTGCAGTGAGCCGAG
ATCGCGACACTGCACTCCAGCCTGGGCGACAGAGTGAGACTGCGTCTCCAAAAAAAAA
AGAAAAAATACGTTATCTATGAAGATTTCCAATTTGATTTCTATTTATCACAAATGGCCACA
GTACTCCTTTGTACTTTACCACATACCATATTGTATTCAGTAATTATTTGTGAATATGTAATT
GATAATATTGTAGGTTTTAGAGAATCCTTGAAAACATGAAAATTTGGTAATGGGGTCTATTT
TGATTATTTATTTATTTATTTATTTATTTTATTTTTGAGACAGAGTCTCGCTCTTGTTGCCCA

GGCTGGAGTGCAGTGGCGCGATCTCGGCTCACTGCAAGCTCCACCTCCCGGGTTCAA
GCGATTCTCCTGCCTCAGCCTCCCAAGTAGCTGGGACTACAGGCACGTGCCACCATGC
CCGGCTAATTTTTTGTATTTTTAGTAGAGGAGGAGTTTCATCTTGTTAGCTAGGATGGTCT
AGATCTCCTGACCTCGTGATCTGCCCGCCTCAGCCTCCCAAAGTGCTGGGATTACAGGT
GTGAGCCACCGTGCCCGGCCATATTTTGATTTAAAATTTAGCAATAATAGATAAAATTTTCA
ATCAACTAAGCCCTTGGGCCAGGGAATGCTATTCCTTAAAAAGTGCTTCTATCAATATAGC
CTCTGACTCATTACTTTGTTAATTTTTAAATTGTATTTCATTCCTGATTAACATTCCCACCCA
GATTATTAATTATACAATCTGTTAACTGTAGAACCTCAAACATGTTGGATTGTACTGTATTTG
TCTGGAAGACACATTTTTAAAACATTGTAATCGCTATAAGAGAAGCACTGGGAAAGAAAG
GAGCTTCTATGCCTGCAGTGCCTGAGGAGCCCTTTAACAGTGTGCCCCGCCCCTAAGC
TACTCATGCAGTCATCCCCATCCCAGTTAGTCAACTTTATTCCAAAAAACTTGGTGTTCCA
AATTTTTCCTTCTCAAAGCCCACAGATCCAAAATTCATCAGCAGTTCCCACAAACGTTAC
CCTCACAATGAATCCAGCCATTTTTCACCCTCTCCAGTGGTACCATCATAGCCCAAGCCG
CCACCATTTCTCACCCCCGGTTAACAGGCCACCCTCCTTCTACCCTTATCCTGCTAGAGT
TTGTTTTATCTACAGTGATCAGAAAGATCAGCCTAAAAGATAATTCTGATCACCACCCTCC
TCTACTCACAACCCGGCCGTGTCTCCCCATTGCCCTCAGTGTAGAAGTCAATGTCCCTT
TGCTGAAATGCAACCTTAGTGAAACTTTCCATGACTAACCTCCTTTAAAATTGCAACCTG
GTCCACCCTTACTCCCCCTTACCCCACTTCTCTTTTTTGCACAGCACTTATTTTACCTTCT
AACATACTGTATAATGTACTCATGTATTGTAATTATTGCTTATCATCCCTCTTTCAGTTGCTT
ATATTTTTCATCAATGTGTACCCAGTGCCTAGGACAATATCTGTCTAGGACAAATGGGTAG
TTATGTGGCTGTAGGCAAGCCATTTAACCTCTCTGTACCTCAGTTACTTTATCTGTATCCA
CTTTGCGGTGTTGTCATGAGGATTAAATCAGATAGCCTATGTGTAGCACCTGGCAGTGAA
TTTATCACCCTGTACTGTAACTGTCTACTTTTCTGTCTCCTCCATTGGACTGTCATTCCCA
GGGGGTTGGGAACTGGGATTTCTTCATTTCTGAGGCATAGAAGTATAGCATAGTGGTTAG
GAGCATGACTTCTGGAGCCAGAGTACATGGGTTTGAATGCTACCACTCACAAGCTGTGT
GGCCATGGAGAAGTTGCCTAACCTCTCCGTGCTTCAGTTTCATCACCCATAAAATGAAG
GTAAGAATAGTACCTGTATTTAAAAGCACCTAGAACAGTTCCTGGCATATAGTGTCAGCTG
TCATCTCTGCATCCTTGTACCTGTCAGAGAGGAGTGTTTATCAAAGGGGCTTCTTGCTGC
CTGTTTCCAAACCAGTCGACAATATACCAATTGCTCCCTAACACATTCTTGTTTGTGCAGA
ACTGAGCTCAATGATAACATTTTTATAGCAACCCTGATCAAGTTTCTTCTCATAATCTCTTA
CACTTTGAGGCCCCTGCAGGGGCCCTCACTCTCCCTAATAAACATTAACCTGAGTAGGG
TGTTTGAGCTCACCATGGCTACATTCTGATGTAAAGAGATATATCCTATACCTGGGCCAAA
TGTAAACAGCCTGGAAAAGTGTTAGGTTAAAAACAAAACAAAATAAATAAATGAATAAATG
CCAGGTGGTTATGAGTGCTATTGAGAAAAATGAAGCCAAGAGGGATATCAGTGATGCAG
GTGGGGGTAAAGAGCTTACAACATAAATGTGGTGTTCCATATTTAAACCTCATTCAACAG
GGAAGATTGGAGCTGAAATGTGAAGGAGTTGTGGGAGTGGAACTACGTGGAAATCTGG
GGGAAAGGTGTTTTGGGTAAAAGAAATAGCAAGTGTTGAGGTCCAGGGGCATGAGTGT

GCTTGATATTTTAGGGAAGAGTAAGGAGACCAGTATAACCAGAGTGAGATGAGACTACAG
AGGTCAGGAGAAAGGGCATGCAGACCATGTGGGATGCTCTAGGACCTAGGCCATGGTA
AAGATGTAGGGTTTTACCCTGATGGAGGTCAGAAGCCATTGGAGGATTCTGAGAAGAGG
AGTGACAGGACTCGCTTTATAGTTTTAAATTATAACTATAAATTATAGTTTTTAAAACAATAG
TTGCCTAACCTCATGTTATATGTAAAACTACAGTTTTAAAAACTATAAATTCCTCATACTGG
CAGCAGTGTGAGGGGCAAGGGCAAAAGCAGAGAGACTAACAGGTTGCTGGTTACTCTT
GCTAGTGCAAGTGAATTCTAGAATCTTCGACAACATCCAGAACTTCTCTTGCTGCTGCCA
CTCAGGAAGAGGGTTGGAGTAGGCTAGGAATAGGAGCACAAATTAAAGCTCCTGTTCAC
TTTGACTTCTCCATCCCTCTCCTCCTTTCCTTAAAGGTTCTGATTAAAGCAGACTTATGCC
CCTACTGCTCTCAGAAGTGAATGGGTTAAGTTTAGCAGCCTCCCTTTTGCTACTTCAGTT
CTTCCTGTGGCTGCTTCCCACTGATAAAAAGGAAGCAATCCTATCGGTTACTGCTTAGTG
CTGAGCACATCCAGTGGGTAAAGTTCCTTAAAATGCTCTGCAAAGAAATTGGGACTTTTC
ATTAAATCAGAAATTTTACTTTTTTCCCCTCCTGGGAGCTAAAGATATTTTAGAGAAGAATT
AACCTTTTGCTTCTCCAGTTGAACATTTGTAGCAATAAGTC (SEQ ID NO: 12).

In yet further embodiments, the promoter and/or the nucleic acid further additionally comprises an enhancer sequence selected from any one or both of the following sequences:

GGGGCTCGCTCGCTCAGTACCTGGAGGCGAGTTCCTGACGCGACTGCGACTCAATCCT
CGCCTGGTGAAGAATATTTTACCTATGACTCACTGAAAATAAAGACGGCTGAGTGACCGT
GTTTGTTCATGTAAACATTGAACAAATATTTATCGGCTTCTGCGATGTGTCCTACTCTTTTA
GTGGAGGAAGACACATTTTATTTATGTATTTAATTTTTCTTTTGAATTTTACATGCGAGTTAT
ACTTAATAAAACTCACTTCAAAATATACCTTCAACAGAAAATCCAGCAACAGTTTCTATTAT
GTTAGTTAAAACAGCCAGTCTTTTCCTTTACTTTTAAAAATTATTCATAAATGTAATTAGTGA
ATGATAATAAACATTGACATCTGATCCACTGCTTTAGGAGTGACACAAATGAAGTTAACTC
AGGCTATTTTCTTTATAATCATTGTGCTATTGTTTTCTTTTTCTTTTCAATTATACTGCTTAAT
ATAGGATTTTGTGGCACCATAGGAGTTGAG (SEQ ID NO: 13).

TCGCCACCACTTGGCTTCCGGCACGTGGGGCAGATGTTTCCATTCCCACGGCGGCAGC
GGAAGAGGGAGGGCCGGGCGCGCCGCGGCTGCTTGCAGTCTCCGCAAGCGGCTACA
TCACAGAGCTCAGCGTGCGGTGTCACAGGCCCCGCGGTCCCGCCCAACAGATGCACC
GAGATGCGCGTGCGCAGAAAGCGTCCCGGGGGTGAGGCTCCCTCCCTCGCTCTCCCT
CTACTCCCGCCCCACTCTCCCCCACTTTCCCCCCTCCACCCACCGCGGCCGTCGGGG
CTCGCTCGCTCAGTACCTGGAGGCGAGTTCCTGACGCGACTGCGACTCAATCCTCGCC
TGGTGAAGAATATTTTACCTATGACTCACTGAAAATAAAGACGGCTGAGTGACCGTGTTT
GTTCATGTAAACATTGAACAAATATTTATCGGCTTCTGCGATGTGTCCTACTCTTTTAGTG
GAGGAAGACACATTTTATTTATGTATTTAATTTTTCTTTTGAATTTTACATGCGAGTTATACT

TAATAAAACTCACTTCAAAATATACCTTCAACAGAAAATCCAGCAACAGTTTCTATTATGTT
AGTTAAAACAGCCAGTCTTTTCCTTTACTTTTAAAAATTATTCATAAATGTAATTAGTGAATG
ATAATAAACATTGACATCTGATCCACTGCTTTAGGAGTGACACAAATGAAGTTAACTCAGG
CTATTTTCTTTATAATCATTGTGCTATTGTTTTCTTTTTCTTTTCAATTATACTGCTTAATATAG
GATTTTGTGGCACCATAGGAGTTGAG (SEQ ID NO: 14).

In other embodiments, the promoter may be the long or short form Stabilin2 promoter, or a variant or functional fragment thereof. Thus, in further embodiments, the promoter may be selected from or may comprise any one of the following sequences:

CGTTTCCATCATGGTTTCCAATAGAATTGAGAATACCATCTATTATTTATTTATTCATTAAATA
TTGATTGTTTCAGCAGCAGAGGGCAGTGGTTGACAGTACTGGCTCTGGAGCCACATAGT
TTGAATACTAGATTTACCACCTATTAGCTGTGTGGCCTTGGATAAATTCCTTAACCTCTCT
GCCTCAGTTTCCTCCTCTGTAAAATGAGAAGAATCATCCTGTTTTACCAAGTACTGTGAG
AATTAAACAACTAAATCCATGGAGAGGCCTAGAATAGTGACTAGCATGTAGTAAGTTTTCA
AAAAGTGATGGCTACTATCCTTAACTGTTTTCATTATTTTGAAGTGCAGGTCTCTGCTCTA
GGAATCAAGGGGTACAGAGAAGATGAGGCTATATTCTTCTGTCCTCAAGGCATTCATAGC
TGGGCATGGGAGACAGACAGATAGATGTGACCTCGGCTTTCATGGCTTTATGTGCAAAA
TTGTGGGAGCATCTTCAGGGTCCTGTGGAGGGGCAGCACAGGAAGGCTTCCTGGAAG
GGGTGGCTTTAGATCTGGAACTTAAAGGGTAAGATTGAACCTACAGGCTCAGTCACTTG
TTACTGAAGTGGCATGGAAAAGAAGAAAGAATGAGTCAAAAACCCACCTGTCAGCATCT
GCAAGGAGACTTTTTTTGCAGTAGAGGTTATCCAGGGTGAAGCCACCACCTCCTGCAAG
GTTTCTGCAACATTCATTCCATACCTGGGACCGTGCTCACTTATGGGCACTCCCCCAACC
CCGCTCGACAGCAAGCTTGGACCTTTGCATTGGCACCAAAAGGCCTGCAGTGTTCCAT
GGCGTGGTTGTGGAAACATTGACAGGGCCGCAGGGCAGCAGTAGGCAGGACTAGAGG
AGGGGGCAGAGTAGAGGAGAGGCAGGACCGTTCTGAGAGCTTCGCAGGCACAAAAAT
CCCGGGCCAAGCCTCTGCCTCAGCCCCTGCAGGATATCCTGTCATTCAGCAGGATATAT
AGACCATTTACAGTGGCCAATTTCACCAAGACTCCTGGATTTGCCATTTTTCCTTTCTGAA
GGCAGGTCTCACCTATCTCCTGGTTCGATCTAGGAAAAAGGAAAGGAAGGGATTTAAAA
GTAAACAGTGAAATGAGAAAGAATTCACTGGGAGTTTATCAAACTAAGTTAAAATAGCTAA
GTCAGCCTGACAGGTGCTTGGCACAGAGAAGGAGCAAATATTTCCTC (SEQ ID NO: 15).

GTAGAGGAGAGGCAGGACCGTTCTGAGAGCTTCGCAGGCACAAAAATCCCGGGCCAA
GCCTCTGCCTCAGCCCCTGCAGGATATCCTGTCATTCAGCAGGATATATAGACCATTTAC
AGTGGCCAATTTCACCAAGACTCCTGGATTTGCCATTTTTCCTTTCTGAAGGCAGGTCTC
ACCTATCTCCTGGTTCGATCTAGGAAAAAGGAAAGGAAGGGATTTAAAAGTAAACAGTGA

AATGAGAAAGAATTCACTGGGAGTTTATCAAACTAAGTTAAAATAGCTAAGTCAGCCTGAC
AGGTGCTTGGCACAGAGAAGGAGCAAATATTTCCTC (SEQ ID NO: 16).

In other embodiments, the promoter may be the vascular endothelial cadherin (VEC) promoter, or a variant or functional fragment thereof. In still other embodiments, the promoter is the intercellular adhesion molecule 2 (ICAM-2) promoter; the Tie2 promoter; or the flk-1 promoter, or any variants or functional fragments thereof. All promoters described herein are known in the art and it will be appreciated that addition, removal or substitution to any of these may be envisaged. For example, use of the F8 promoter to drive FVIII expression is described in Merlin *et al.* (2019), use of the VEC promoter to drive FVIII expression is described in Merlin et al. ((2017) Mol. Ther., 25(8):1815-1830, doi: https://doi.org/10.1016/i.vmthe.2017.04.029) and use of the ICAM-2, Tie2 and flk-1 promoters are described in De Palma et al. ((2003) Hum. Gene. Ther., 14(12):1193-206, doi: https://doi.org/10.1089/104303403322168028), the promoter sequences of which and their uses are hereby specifically incorporated by reference in their entirety.

Use of a tissue- or organ-specific promoter which is the endogenous promoter for the gene to be expressed has the advantage not only of restricting expression to specific cells, but also of providing near-endogenous levels of expression, i.e. neither over expression nor under expression compared to physiological levels. Such expression levels may therefore also be referred to as "physiological expression levels". In one embodiment, the endogenous/ physiological expression level achieves replacement of a defective or non-expressed gene/ protein, such that said expression level may be considered therapeutic. In addition, the location or cell-type expressing the nucleic acid/transgene may be considered therapeutic. Thus, in one embodiment the expression of the sequence comprised in the nucleic acid is both therapeutic in its level and in terms of the tissue/organ/cells from which it is expressed. Such therapeutic expression may also be referred to as "therapeutically relevant". In one embodiment, the nucleic acid provided by the GP64-LVs herein to liver endothelial cells, in particular LSECs, is expressed at a therapeutically relevant level. In a further embodiment, the sequence/transgene encoding factor VIII provided by the GP64-LVs herein is expressed at a therapeutically relevant level, in particular in liver endothelial cells (e.g. LSECs) transduced by the GP64-LVs. In a still further embodiment, the therapeutically relevant expression level is about 5% to about 50% of the physiological level. In one embodiment, the therapeutically relevant expression level is about 10% to about 20%, to about 30% or to about 40% of the physiological level. In another embodiment, the therapeutically relevant expression level is about 10% to about 20% of the physiological level. In a further embodiment, the therapeutically relevant expression level is about 10%, about 15% or about 20% of the physiological level.

In a still further aspect of the invention, there is provided a lentivirus vector particle pseudotyped with the baculovirus-derived GP64 envelope protein and comprising a nucleic acid, said nucleic acid comprising the long or short form endogenous human factor VIII (F8) promoter operably linked to a coding sequence. As demonstrated herein, such GP64-LVs have been shown to transduce endothelial cells, in particular liver endothelial cells and more particularly LSECs, and drive expression of a transgene/nucleic acid coding for a gene of interest is said cells. Thus, in some embodiments the nucleic acid may further comprise a coding sequence for a gene for liver regeneration, or a gene defective or deficient in a liver disease or haemophilia, such as a coagulation and/or clotting factor. In particular embodiments, the nucleic acid comprises a factor VIII coding sequence, optionally a human codon-optimised factor VIII transgene as described hereinbefore.

### In Vitro, In Vivo, Ex Vivo and Therapeutic Methods & Uses

In some embodiments, the methods provided herein are performed *in vitro. In vitro* may include, without limitation, in culture, such as a cell culture. Such cultures may be of cell lines, in particular immortalised cell lines, such as mammalian cell lines, as well as cells obtained from a subject, i.e. which are ex *vivo.* One particular example of cells for transduction *in vitro,* such as ex *vivo,* include blood outgrowth endothelial cells (BOECs). The use of BOECs as a vehicle to deliver a gene therapy for haemophilia A *in vivo* has previously been reported (Lin et al. (2002) Blood, 99(2):457-462, doi: https://doi.org/10.1182/blood.V99.2.457). BOECs are mature endothelial cells that have a high expansion capacity *in vitro,* a stable phenotype and the ability to expand *in vivo* with no apparent toxicity. BOEC-based gene therapy (i.e. where BOECs are transduced *in vitro* and then administered to a subject) alleviates any safety concerns that may be associated with administration of a vector directly *in vivo* and since they may be derived from the subject in need of treatment, would present a low risk of immune response, potentially allowing multiple administrations for chronic disease. Furthermore, since BOECs are mature cells, no adverse effects due to their possible differentiation following administration are expected. Thus, in one embodiment the methods may be performed in an *in vitro* culture. In a further embodiment, use of the GP64-LVs, including CD47hi GP64-LVs, is *in vitro,* such as in an *in vitro* culture. In a yet further embodiment, the methods may be performed ex *vivo,* such as in a culture of ex *vivo* cells. In a still further embodiment, use of the GP64-LVs, including CD47hi GP64-LVs, is ex *vivo,* such as in an ex *vivo* culture. Cells may be obtained from a subject suffering from a disease or disorder for which administration of a gene therapy is desired, i.e. a subject in need of gene therapy. Alternatively or additionally, cells may be obtained from a subject in need of or undergoing transplant treatment, in particular liver transplant. Still alternatively, cells may be obtained from a healthy subject, such as donor of a tissue, organ or cells for transplant.

In further preferred embodiments, the methods provided herein may be performed *in vivo.* Such *in vivo* embodiments for use of the GP64-LV described herein will be appreciated to provide the advantage of specifically targeting transduction to the desired target cells, with restricted tropism for liver endothelial cells (in particular LSECs) and against other cell types, such as hepatocytes, Kupffer cells (KCs), liver macrophages and other haematopoietic cells. Furthermore, by providing restricted tropism for liver endothelial cells (in particular LSECs) over non-liver endothelium, the number of lentiviral vector particles available to transduce the desired target liver endothelial cells is increased by reducing the number of lentiviral vector particles which may be 'soaked up' by these non-target cells, as described hereinbefore.

Thus, in a further aspect of the invention, there is provided a method of treating a subject in need thereof, the method comprising administering to the subject a lentiviral vector particle as described herein. In one embodiment, there is provided a method of treating a subject in need of liver regeneration or suffering from a liver disease or haemophilia, the method comprising administering to the subject a lentiviral vector particle pseudotyped with the baculovirus-derived GP64 envelope protein and comprising a nucleic acid, said nucleic acid comprising a coding sequence for a gene for liver regeneration, or a gene defective or deficient in a liver disease or haemophilia. In a certain embodiment, the method is for treating a subject suffering from haemophilia, the method comprising administering to the subject a lentiviral vector particle pseudotyped with the baculovirus-derived GP64 envelope protein and comprising a nucleic acid encoding a gene defective or deficient in haemophilia. Thus, in particular embodiments a lentiviral vector particle pseudotyped with the baculovirus-derived GP64 envelope protein and comprising a nucleic acid as described herein is administered. The administered GP64-LVs may comprise, such as further comprise, CD47, or a functional fragment thereof, on their surface. Thus, in another embodiment CD47hi GP64-LVs as described herein may be administered. Such administration may be systemically, e.g. enteral or parenteral, such as via intravenous infusion, or locally, such as directly into the tissue or organ to be treated, e.g. by topical administration, such as directly into the liver. As will be appreciated from the disclosures herein, due to the high tropism/selectivity achieved by the methods and GP64-LVs of the invention, administration may be systemic without significant off-target effects, such as transduction of tissues or organs other than the liver, or transduction of cells other than LSECs. As such, in certain embodiments the lentiviral vector particle transduces liver sinusoidal endothelial cells (LSECs) in the subject, i.e. *in vivo.*

In a further aspect, there is provided a lentiviral vector particle as described herein for use in the transduction of liver endothelial cells *in vivo.* In some embodiments, the liver endothelial cells are liver sinusoidal endothelial cells (LSECs) as described herein. Thus, in particular embodiments a lentivirus vector particle pseudotyped with the baculovirus-derived GP64 envelope protein (GP64-LV) for use in transducing liver endothelial cells (in particular LSECs) is provided. The GP64-LV may comprise, such as further comprise, CD47, or a functional fragment thereof, on its surface. Thus, in another embodiment a CD47hi GP64-LV for use in transducing liver endothelial cells (in particular LSECs) is provided. Such transduction may be for the treatment of a disease or disorder for which gene therapy is desired, i.e. the subject in need of treatment is in need of gene therapy. For example, transduction may be for liver regeneration, or for the treatment of a liver disease or haemophilia. Thus, according to these embodiments transduction is *in vivo.*

In a further particular embodiment, the subject is suffering from haemophilia. In a yet further embodiment, the haemophilia is haemophilia A. Thus, in some embodiments of both the methods of treatment and therapeutic uses herein, the treatment or therapy is for haemophilia. In a particular embodiment, the treatment or therapy is for haemophilia A. Thus, in a yet further embodiment the transduction is for the treatment of haemophilia A. In a still further embodiment, the treatment is for haemophilia and the lentiviral vector particle comprises a nucleic acid encoding a gene defective or deficient in haemophilia. In one embodiment, the gene defective or deficient in haemophilia encodes for a coagulation and/or clotting factor.

Thus, in certain embodiments the nucleic acid comprises a factor VIII coding sequence. In a further embodiment, the factor VIII coding sequence is a human codon-optimised factor VIII transgene as described hereinbefore. In a yet further embodiment, the nucleic acid further comprises a liver-specific promoter as described hereinbefore. Thus, in a still further embodiment, the nucleic acid further comprises an endothelial cell-specific promoter, such as an LSEC-specific promoter. In a particular embodiment, the promoter is the endogenous human factor VIII (F8) promoter (e.g. the long or short form F8 promoter as described herein).

Thus, according to one aspect of the invention there is provided a method of treating a subject suffering from haemophilia, the method comprising administering to the subject a lentiviral vector particle pseudotyped with the baculovirus-derived GP64 envelope protein and comprising a nucleic acid encoding a gene defective or deficient in haemophilia. In a further aspect, there is provided a lentiviral vector particle pseudotyped with the baculovirus-derived GP64 envelope protein and comprising a nucleic acid encoding a gene defective or deficient in haemophilia for use in the transduction of liver sinusoidal endothelial cells (LSECs) *in vivo,* wherein the transduction is for the treatment of haemophilia.

According a still further aspect, there is provided a pharmaceutical composition comprising the lentivirus vector particle described herein, optionally in combination with one or more pharmaceutically acceptable excipients, carriers and/or diluents. Generally, pharmacologically appropriate excipients, carriers or diluents will be utilised. Typically, these include aqueous or alcoholic/aqueous solutions, emulsions or suspensions, including saline and/or buffered media. Parenteral vehicles include sodium chloride solution, Ringer's dextrose, dextrose and sodium chloride and lactated Ringer's. Suitable physiologically-acceptable adjuvants, if necessary to keep a composition comprising the targeting moiety specific for a tissue or organ as defined herein in a discrete location (e.g. within a tissue or organ of interest), may be chosen from thickeners such as carboxymethylcellulose, polyvinylpyrrolidone, gelatine and alginates. Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

Thus, in another aspect, there is provided a use of the GP64 pseudotyped lentiviral vector particle described herein in the manufacture of a medicament. Such medicaments may be used in any of the methods or therapeutic uses described hereinbefore, in particular for liver regeneration, or for the treatment of a liver disease or haemophilia. More particularly, the medicaments may be used for the treatment of haemophilia. Thus, in one embodiment the methods of providing a gene for liver regeneration, or for the treatment of a liver disease or haemophilia herein comprise administering a medicament comprising the GP64 pseudotyped lentiviral vector particle as described herein. In another embodiment, there is provided the medicament for use in the treatment of haemophilia. In a further embodiment, there is provided the use of GP64-LV, including CD47hi GP64-LV, described herein in the manufacture of a medicament for the treatment of haemophilia, such as haemophilia A.

It will be appreciated that references herein to a patient or subject relate equally to animals and humans and that the invention will find utility in veterinary treatment of any of the above mentioned diseases, disorders and conditions which are also present in said animals. Thus, in some embodiments the subject is a human subject. In other embodiments, the subject is a non-human animal, such as a non-human primate or a rodent (e.g. a mouse or rat).

It will also be appreciated that references herein to "treatment" and "amelioration" include such terms as "prevention", "reversal" and "suppression". Furthermore, such references include administration or use of the GP64 pseudotyped lentivirus vector particle or composition comprising said GP64-LV as defined herein prior to the onset of the disease or disorder, e.g. wherein the subject is at risk of the disease or disorder. Administration or use of the GP64-LV or composition as defined herein may also be anticipated after the induction event of the injury, damage, disease or disorder, either before clinical presentation of said disease or disorder, or after symptoms manifest. Such references may also include administration or use of the GP64-LV as defined herein prior to or following transplant.

Unless defined otherwise, all technical and scientific terms used herein have the meaning commonly understood by a person skilled in the art to which this invention belongs. As may be used herein, the term "about" includes up to and including 10% greater and up to and including 10% lower than the value specified, suitably up to and including 5% greater and up to and including 5% lower than the value specified, especially the value specified. The term "between" as may be used herein includes the values of the specified boundaries.

Throughout the specification and the claims which follow, unless the context requires otherwise, the word "comprise", and variations thereof such as "comprises" and "comprising", will be understood to imply the inclusion of a stated integer, step, group of integers or group of steps but not to the exclusion of any other integer, step, group of integers or group of steps.

In addition, as used herein and the appended claims, the singular forms "a", "an" and "the" include plural referents, and *vice versa,* unless the content clearly dictates otherwise. Thus, for example references to "a lentivirus vector particle" include two or more such particles.

It will be understood that all embodiments described herein may be applied to all aspects of the invention and *vice versa,* and such combinations would be readily apparent from the description provided herein and to those skilled in the art.

Other features and advantages of the present invention will be apparent from the description provided herein. It should be understood, however, that the description and the specific examples while indicating preferred embodiments of the invention are given by way of illustration only, since various changes and modifications will become apparent to those skilled in the art.

### CLAUSES

A set of clauses defining the invention, its aspect and embodiments is as follows:
1. A method of transducing liver endothelial cells with a nucleic acid, wherein the nucleic acid is transduced by means of a lentiviral vector particle pseudotyped with the baculovirus-derived GP64 envelope protein and comprising said nucleic acid.
2. The method of clause 1, wherein the nucleic acid comprises a liver-specific promoter operably linked to a coding sequence.
3. The method of clause 2, wherein the promoter is an endothelial cell-specific promoter, such as an LSEC-specific promoter.
4. The method of clause 3, wherein the promoter is selected from: the long or short form endogenous human factor VIII (F8) promoter; the long or short form Stabilin2 promoter; the vascular endothelial cadherin (VEC) promoter; the intercellular adhesion molecule 2 (ICAM-2) promoter; the Tie2 promoter; or the flk-1 promoter.
5. The method of clause 3 or clause 4, wherein the promoter is the endogenous human factor VIII (F8) promoter.
6. The method of any one of clauses 1 to 5, wherein the nucleic acid comprises a coding sequence for a gene for liver regeneration, or a gene defective or deficient in a liver disease or haemophilia, such as a coagulation and/or clotting factor.
7. The method of clause 6, wherein the nucleic acid comprises factor VIII coding sequence.
8. The method of clause 7, wherein the factor VIII coding sequence is a human codon-optimised factor VIII transgene.
9. The method of any one of clauses 1 to 8, wherein the method is performed *in vitro, in vivo* or ex *vivo,* in particular *in vivo.*
10. A method of treating a subject in need of liver regeneration or suffering from a liver disease or haemophilia, the method comprising administering to the subject a lentiviral vector particle pseudotyped with the baculovirus-derived GP64 envelope protein and comprising a nucleic acid, said nucleic acid comprising a coding sequence for a gene for liver regeneration, or a gene defective or deficient in a liver disease or haemophilia.
11. The method of clause 10, wherein the nucleic acid comprises a coding sequence for a gene defective or deficient in haemophilia, such as haemophilia A.
12. The method of clause 11, wherein the gene defective or deficient in haemophilia encodes for a coagulation and/or clotting factor.
13. The method of any one of clauses 10 to 12, wherein the nucleic acid comprises a factor VIII coding sequence.
14. The method of clause 13, wherein the factor VIII coding sequence is a human codon-optimised factor VIII transgene.
15. The method of any one of clauses 10 to 14, wherein the nucleic acid comprises a liver-specific promoter operably linked to the coding sequence
16. The method of clause 15, wherein the promoter is an endothelial cell-specific promoter, such as an LSEC-specific promoter.
17. The method of claim 15 or claim 16, wherein the promoter is selected from: the long or short form endogenous human factor VIII (F8) promoter; the long or short form Stabilin2 promoter; the vascular endothelial cadherin (VEC) promoter; the intercellular adhesion molecule 2 (ICAM-2) promoter; the Tie2 promoter; or the flk-1 promoter.
18. The method of any one of clauses 15 to 17, wherein the promoter is the endogenous human factor VIII (F8) promoter.
19. The method of any one of clauses 10 to 18, wherein the lentiviral vector particle transduces liver endothelial cells in the subject.
20. The method of clause 19, wherein the liver endothelial cells are liver sinusoidal endothelial cells (LSECs).
21. A lentiviral vector particle pseudotyped with the baculovirus-derived GP64 envelope protein for use in the transduction of liver endothelial cells with a nucleic acid *in vivo.*
22. The lentivirus vector particle for use of clause 21, wherein the nucleic acid comprises a liver-specific promoter operably linked to a coding sequence.
23. The lentivirus vector particle for use of clause 22, wherein the promoter is an endothelial cell-specific promoter, such as an LSEC-specific promoter.
24. The lentivirus vector particle for use of clause 22 or clause 23, wherein the promoter is selected from: the long or short form endogenous human factor VIII (F8) promoter; the long or short form Stabilin2 promoter; the vascular endothelial cadherin (VEC) promoter; the intercellular adhesion molecule 2 (ICAM-2) promoter; the Tie2 promoter; or the flk-1 promoter.
25. The lentivirus vector particle for use of any one of clauses 22 to 24, wherein the promoter is the endogenous human factor VIII (F8) promoter.
26. The lentivirus vector particle for use of any one of clauses 21 to 25, wherein the transduction is for liver regeneration, or for the treatment of a liver disease or haemophilia, such as haemophilia A.
27. The lentivirus vector particle for use of clause 26, wherein the nucleic acid comprises a coding sequence for a gene for liver regeneration, or a gene defective or deficient in a liver disease or haemophilia.
28. The lentivirus vector particle for use of clause 27, wherein the gene defective or deficient in haemophilia encodes for a coagulation and/or clotting factor.
29. The lentivirus vector particle for use of any one of clauses 21 to 28, wherein the nucleic acid comprises a factor VIII coding sequence.
30. The lentivirus vector particle for use of clause 29, wherein the factor VIII coding sequence is a human codon-optimised factor VIII transgene.
31. A lentivirus vector particle pseudotyped with the baculovirus-derived GP64 envelope protein and comprising a nucleic acid, said nucleic acid comprising the long or short form endogenous human factor VIII (F8) promoter operably linked to a coding sequence.
32. The lentivirus vector particle of clause 31, wherein the nucleic acid further comprises a coding sequence for a gene for liver regeneration, or a gene defective or deficient in a liver disease or haemophilia.
33. The lentivirus vector particle of clause 32, wherein the gene defective or deficient in haemophilia is a coagulation and/or clotting factor.
34. The lentivirus vector particle of any one of clauses 31 to 33, wherein the nucleic acid comprises a factor VIII coding sequence.
35. The lentivirus vector particle of clause 34, wherein the factor VIII coding sequence is a human codon-optimised factor VIII transgene.
36. The method, lentivirus vector particle for use or lentivirus vector particle of any preceding clause, wherein the liver endothelial cells are liver sinusoidal endothelial cells (LSECs) and/or LSECs are transduced.
37. The method, lentivirus vector particle for use or lentivirus vector particle of any preceding clause, wherein the GP64 envelope protein comprises an amino acid sequence of SEQ ID NO: 1, or a variant or functional fragment thereof.
38. The method, lentivirus vector particle for use or lentivirus vector particle of clause 37, wherein the GP64 envelope protein consists of an amino acid sequence of SEQ ID NO: 1.
39. The method, lentivirus vector particle for use or lentivirus vector particle of clause 37, wherein the variant or functional fragment promotes the binding and fusion of the viral vector to the cell membrane in a similar manner or to a similar extent to full length GP64 and/or promotes viral entry into a target cell.
40. The method, lentivirus vector particle for use or lentivirus vector particle of any preceding clause, wherein the GP64 envelope protein comprises an amino acid sequence encoded by the nucleotide sequence of SEQ ID NO: 2, or a variant or functional fragment thereof.
41. The method, lentivirus vector particle for use or lentivirus vector particle of clause 37, wherein the GP64 envelope protein consists of an amino acid sequence encoded by SEQ ID NO: 2.
42. The method, lentivirus vector particle for use or lentivirus vector particle of clause 37, wherein the GP64 envelope protein comprises a functional amino acid sequence encoded by a fragment of SEQ ID NO: 2, or a variant thereof.
43. The method, lentivirus vector particle for use or lentivirus vector particle of any preceding clause, wherein the lentiviral vector particle further comprises high levels of CD47, or a variant or functional fragment thereof, on its surface.
44. The method, lentivirus vector particle for use or lentivirus vector particle of clause 43, wherein the CD47 protein comprises an amino acid sequence of SEQ ID NO: 3, or a variant or functional fragment thereof.
45. The method, lentivirus vector particle for use or lentivirus vector particle of clause 44, wherein the CD47 protein consists of an amino acid sequence of SEQ ID NO: 3.
46. The method, lentivirus vector particle for use or lentivirus vector particle of clause 44, wherein the variant or functional fragment retains the phagocytosis inhibitor function of CD47 and/or inhibits or reduce phagocytosis compared to a lentivirus vector particle not comprising high levels of CD47, or a variant or fragment thereof, on its surface.
47. The method, lentivirus vector particle for use or lentivirus vector particle of clause 44 or clause 46, wherein the CD47 variant or functional fragment comprises the extracellular domain of CD47.
48. The method, lentivirus vector particle for use or lentivirus vector particle of clause 47, wherein the CD47 variant or functional fragment comprises an amino acid sequence of SEQ ID NO: 4.
49. The method, lentivirus vector particle for use or lentivirus vector particle of any preceding clause, wherein the CD47 protein comprises an amino acid sequence encoded by the nucleotide sequence of SEQ ID NO: 5, or a variant or functional fragment thereof.
50. The method, lentivirus vector particle for use or lentivirus vector particle of clause 49, wherein the CD47 protein consists of an amino acid sequence encoded by SEQ ID NO: 5.
51. The method, lentivirus vector particle for use or lentivirus vector particle of clause 49, wherein the CD47 protein comprises a functional amino acid sequence encoded by a fragment of SEQ ID NO: 5, or a variant thereof.
52. The method, lentivirus vector particle for use or lentivirus vector particle of clause 51, wherein the CD47 variant or functional fragment comprises an amino acid sequence encoded by the nucleotide sequence of SEQ ID NO: 6.
53. The method, lentivirus vector particle for use or lentivirus vector particle of clause 52, wherein the amino acid sequence encoded by SEQ ID NO: 6 is the extracellular domain of CD47.
54. The method, lentivirus vector particle for use or lentivirus vector particle of clause 53, wherein the CD47 variant or functional fragment comprises the amino acid of SEQ ID NO: 4.
55. The method, lentivirus vector particle for use or lentivirus vector particle of any preceding clause, wherein the lentiviral vector particle comprises low levels of MHC-I on its surface or is MHC-I free.
56. The method, lentivirus vector particle for use or lentivirus vector particle of clause 55, wherein the lentiviral vector particle comprises low levels of MHC-I on its surface or is MHC-I free, and comprises high levels of CD47, or a variant or functional fragment thereof, on its surface.

The invention will now be described using the following, non-limiting examples:

### EXAMPLES

### Materials and Methods

### Plasmid Construction

Plasmid pCCLsin.cPPT.PGK.GFP and pCCLsin.cPPTETFIX.142T were previously described. Packaging plasmid encoding for GP64 was previously described (Schauber *et al.* (2004)). Plasmid pCCLsin.cPPT.F8.coFVIII.142T was constructed by standard cloning techniques, by exchanging the gene synthesized human FVIII cDNA (Milani *et al.* (2022)) with factor IX (FIX) into the previously described pCCLsin.cPPT.ET.canineFIX.142T (Nhel-Sall digestion) and then exchanging the ET promoter with the F8 promoter (Merlin *et al.* (2019)). GFP-pp60Src fusion construct was previously described (Milani *et al.* (2019)).

### Vector Production

Third-generation self-inactivating (SIN) LV were produced by calcium phosphate transient transfection into 293T cells. 293T cells were transfected with a solution containing a mix of the selected LV genome transfer plasmid, the packaging plasmids pMDLg/pRRE and pCMV.REV, pMD2.G or pBA-AcMNPV-gp64 (Schauber *et al.* (2004)) and pAdVantage (Promega), as previously described (Milani *et al.* (2019)). Briefly, medium was changed 14-16 hours after transfection and supernatant was collected 30 hours after medium change. LV-containing supernatants were sterilized through a 0.22µm filter (Millipore), transferred into sterile poliallomer tubes (Beckman) and centrifuged at 20,000 g for 120 min at 20° C (Beckman Optima XL-100K Ultracentrifuge). For FVIII transgene encoding LV, before centrifugation, LV-containing 0.22µm-filtered supernatants were pre-concentrated 4 times with Vivaflow 200 Tangential Flow Filtration Cassette 100 kDa (Sartorius), according to manufacturing instructions. LV pellet was dissolved in the appropriate volume of PBS to allow 500-1000X concentration. To produce fluorescent virions, 15µg/plate of pMAX.GFP^{pp60Src} plasmid were added to plasmid mix in absence of genome-encoding plasmid to produce empty LV particles. Bald-LV (LV lacking envelope protein) were produced in the absence of envelope-encoding packaging plasmid.

### LV Titration

For LV titration, 1×10⁵ 293T or Huh7 cells were transduced with serial LV dilutions in the presence of polybrene (8µg/ml). For LV-GFP, cells were analysed by flow cytometry using a FACSCanto analyser (BD Biosciences), equipped with DIVA Software, 5-7 days after transduction and infectious titer, expressed as transducing units (TU)/mL, was calculated using the formula TU/mL = ((% GFP+ cells/100)x100,000x(1/dilution factor)). For all other LV, genomic DNA Genomic DNA (gDNA) was extracted 14 days after transduction, using Maxwell 16 Cell DNA Purification Kit (Promega), following manufacturer's instructions. Vector copy number (VCN) was determined by ddPCR, starting from 5-20ng of template gDNA using primers (HIV fw: 5'-T ACTGACGCTCTCGCACC-3'; HIV rv: 5'-TCTCGACGCAGGACTCG-3') and a probe (FAM 5'-ATCTCTCTCCTTCTAGCCTC-3') designed on the primer binding site region of LV. The amount of endogenous DNA was quantified by a primers/probe set designed on the human telomerase gene (Telo fw: 5'-GGCACACGTGGCTTTTCG-3'; Telo rv: 5'-GGTGAACCTCGTAAGTTTATGCAA-3'; Telo probe: VIC 5'-TCAGGACGTCGAGTGGACAC GGTG-3' TAMRA) or the human GAPDH gene (Applied Biosystems HS00483111_cm). The PCR reaction was performed with each primer (900nM) and the probe (250nM, 500nM for Telo) following manufacturer's instructions (Biorad), read with QX200 reader and analyzed with QuantaSoft software (Biorad). Infectious titer, expressed as TU/mL, was calculated using the formula TU/mL = (VCNx100,000x(1/dilution factor). LV physical particles were measured by HIV-1 Gag p24 antigen immunocapture assay (Perkin Elmer) following manufacturer's instructions. LV specific infectivity was calculated as the ratio between infectious titer and physical particles.

### Vector Copy Number (VCN) Determination

For experiments with primary hepatocytes, DNA was extracted using Maxwell 16 Tissue DNA Purification Kit (Promega), following manufacturer's instructions. For mice experiments, DNA was extracted from whole liver or whole spleen samples using Maxwell 16 Tissue DNA Purification Kit (Promega), DNA was extracted from fractionated/sorted liver cells using DNeasy Blood & Tissue Kit (Qiagen) or QlAamp DNA Micro Kit (Qiagen), according to cell number. VCN was determined in Huh7 samples as described above (see "LV Titration"). VCN in murine DNA line was determined by ddPCR, starting from 5-20ng of template gDNA using a primers/probe set designed on the primer binding site region of LV (see "LV Titration" above). For primary hepatocytes, because cells do not divide in culture, VCN was determined using an ad hoc ddPCR (QX200 EvaGreen Digital PCR Supermix, Bio-Rad), which selectively amplifies the reverse transcribed vector genome (both integrated and non-integrated) discriminating it from plasmid carried over from the transient transfection (RT-LV; ΔU3 fw: 5'-TCACTCCCAACGAAGACAAGATC-3', gag rv: 5'-GAGTCCTGCGTCGAGAGAG-3'; Matrai et al. (2011) Hepatology, 53(5):1696-1707, doi: https://doi.org/10.1002/hep.24230). The amount of endogenous DNA was quantified by primers set designed on the human telomerase gene, as above, or the murine Sema3A gene, as described below. The amount of endogenous murine DNA was quantified by a primers/probe set designed on the murine sema3a gene (Sema3A fw: 5'-ACCGATTCCAGATGATTGGC-3'; Sema3A rv: 5'-TCCATATTAATGCAGTG CTTGC-3'; Sema3A probe: HEX 5'-AGAGGCCTGTCCTGCAGCTCATGG-3' BHQ1). The PCR reaction was performed with each primer (900nM, 150nM for RT-LV primers) and the probe (250nM) following manufacturer's instructions (Biorad), read with QX200 reader and analyzed with QuantaSoft software (Biorad).

### Cell Cultures and In Vitro Experiments

293T cell lines were maintained in Iscove's modified Dulbecco's medium (IMDM, Sigma) supplemented with 10% fetal bovine serum (FBS, FetalClone II, HyClone, Euroclone), 4mM glutamine (Lonza), penicillin and streptomycin 100 international units (IU)/mL (Lonza).

Immortalized human liver sinusoidal endothelial cells (immLSEC) were purchased from Innoprot (P10652-IM). Cells were cultured on Collagen Type 1-coated flasks (Greiner Bio-One) using MCDB 131 medium (Gibco, Life Technologies) containing proprietary supplements (REF: Olgasi 2021). Cells were plated at a density of 10⁴ cells/cm² and transduced after 8 hours. Fresh medium was added to the cells after 16 hours. immLSEC transduced with a PGK.GFP LV at MOI of 1, 5 and 10, were collected for flow cytometry analysis 72 hours after transduction to determine GFP expression using a FACSCanto analyzer (BD Biosciences), equipped with DIVA Software. For cells transduced with LV-pF8.FVIII, an MOI of 5 and 10 were used and FVIII-containing supernatants were collected after 72 hours and hFVIII concentration was assessed by ELISA (see "FVIII assays"). immLSECs were cultured for 10 days before gDNA isolation and VCN determination.

### RNA Extraction and ddPCR

RNA extraction was performed using RNeasy Plus Micro Kit (Qiagen), according to manufacturer's instructions and reverse transcribed using the SuperScript IV VILO kit (11766050; ThermoFisher Scientific). LV gene expression was assessed by ddPCR starting from 10-25ng of template cDNA using a primers/probe set designed on the WPRE region of LV (WPRE: primer fw 5'-GGCTGTTGGGCACTGACAAT-3'; primer rv 5'-ACGTCCCG CGCAGAATC-3'; probe FAM 5'-TTTCCTTGGCTGCTCGCCTGTGT-3' NGB). HPRT was used as reference gene (dMmuCPE5095493, Biorad). The PCR reaction was performed with each primer (900nM) and the probe (250nM) following manufacturer's instructions (Biorad), read with QX200 reader and analysed with QuantaSoft software (Biorad).

### Mice Experiments

Founder *F8*^{tm1Kaz} Tg(Alb-F8*R593C)T4Mcal/J mice (referred to as HemoA-R593C) were obtained from The Jackson Laboratories (stock #017706). C57BL/6 and NOD mice were purchased from Charles River Laboratories. All mice were maintained in specific pathogen-free conditions. Vector administration was carried out in male and female adult (7-10 weeks old) mice by tail-vein injection (250-500µL/mouse), while in newborns by temporal vein injection (25-30µL/mouse). Mice were bled from the retro-orbital plexus using capillary tubes and blood was collected into 0.38% sodium citrate buffer, pH 7.4. Mice were euthanized by CO₂ inhalation at the scheduled times. All animal procedures were performed according to protocols approved by the Institutional Animal Care and Use Committee.

### Fractionation and Sorting of Liver Cell Sub-Populations

The mouse liver was perfused (15mL/min) via the inferior vena cava with 12.5mL of the following solutions at subsequent steps: 1) PBS EDTA (0.5mM), 2) HBSS (Hank's balanced salt solution, Gibco) and HEPES (10mM), 3) HBSS-HEPES 0.03% Collagenase IV (Sigma). The digested mouse liver tissue was harvested, passed through a 100µm cell strainer (BD Biosciences) and processed into a single-cell suspension. This suspension was subsequently centrifuged three times (30, 25 and 20 g, for 3 minutes, at room temperature) to obtain hepatocytes-containing pellets. The nPC-containing supernatant was centrifuged (650xg, 7 minutes, at room temperature) and recovered cells were loaded onto a 30/60% Percoll (Sigma) gradient (1,800xg, for 15 minutes at room temperature). nPC interface was collected and washed twice. The nPC were subsequently incubated with the following monoclonal antibodies: e-fluor 450-conjugated anti-CD45 (30-F11, e-Bioscience), Allophycocyanin (APC)-conjugated anti-CD31 (MEC13.3, BD Biosciences), phycoerythrin (PE)-conjugated F4/80 (CI:A3-1, Biorad), PE-Cy5-conjugated anti-CD45R/B220 (from BD Biosciences), PE-Cy7-conjugated anti-CD11c (N418, e-Bioscience). nPC subpopulations (LSEC, KC, pDC) were sorted by FACS, BD FACSAria^{™} Fusion Cell Sorter (BD Biosciences); the nPC contaminating the PC suspension, were removed by FACS excluding cells labelled by APC-conjugated anti-CD31/anti-CD45 cocktail, thus obtaining sorted hepatocytes (Hep) using BD FACSMelody^{™} Cell Sorter (BD Biosciences) or MoFlo Astrios EQ Cell Sorter (Beckman Coulter).

### Haemostasis Challenge

Mice were anesthetized and the first finger of right foot was cut at the base. Mice were then returned to their cage and monitored for survival for 24 hours.

### Immunofluorescence Imaging

Livers were harvested from mice, washed briefly in PBS and fixed 4 hours in PBS 4% paraformaldehyde (PFA), then washed again briefly in PBS before being stored at least 24 hours in 30% sucrose 0.02% sodium azide in H₂O. Livers were then frozen in OCT (optimal cutting temperature) compound (Killik, Bio Optica) and slices 5 to 20µm thick were cut at cryostat (Histo-line MC5050), placed on Superfrost^{®} Plus microscope slides (Thermo Scientific) and stored at -80°C. All the staining steps were performed protected from light to preserve endogenous fluorescence. Slides were thawed at room temperature for at least 2 hours, then washed 3 times 5 minutes in PBS 0.1% X-Triton. Edges were drawn around the tissue using immunostaining pap pen (Sigma-Aldrich) to contain staining solutions. Blocking step was performed using PBS 0.1% X-Triton 1% bovine serum albumin (BSA) 5% FBS for 1 hour at room temperature in a humid chamber. Blocking solution was then substituted with mix containing primary antibodies in blocking solution and incubation was carried on for 12-16 hours at 4°C in a humid chamber. Primary antibodies mix solution was removed and slides were washed 3 times 5 minutes in PBS 0.1% X-Triton. Secondary antibodies were diluted in blocking solution together with Hoechst (Invitrogen, 1:20000) and incubated for 1 hour at room temperature in a humid chamber. Slides were then washed in PBS and coverslip was added using Fluoromount-G (Invitrogen) as mounting medium. Slides were dried for 16 hours at room temperature or for longer at 4°C, and stored at 4°C before acquisition. The following antibodies were used for immunofluorescence staining:
Anti-GFP chicken, polyclonal (Abcam, 1:200)
Anti-F4/80 rat, clone CI:A3-1 (Abcam, 1:200)
Anti-Lyve1 rabbit, clone (Novus Biological, 1:250)
Anti-Rabbit IgG Alexa Fluor (AF)647 donkey, polyclonal (Invitrogen, 1:1000)
Anti-Chicken IgYAF488 goat, polyclonal (Invitrogen, 1:1000)
Anti-Rat IgG AF561 goat, polyclonal (Invitrogen, 1:1000)

Images were acquired using confocal microscope Mavig Rs-G4 at 20X magnification. Images were analyzed using Image J or MATLAB software. A custom written MATLAB pipeline was used to analyse the images. Briefly, for all the channels (KC, LSEC, GFP+ and nuclei) black and white masks of the cells/nuclei are extracted by background subtraction and then applying a fixed threshold. A mask of the tissue is obtained similarly on the image calculated as max projection across all the channels. Then combining with logical operations the masks of the different cell kinds and the tissue, it is possible to estimate the fraction of area occupied by the different cell types as well as the fraction of GFP positive cells within each population.

### Immunohistochemistry Imaging

Livers were perfused from the inferior vena cava with PBS EDTA 0.5mM to remove blood, then harvested and fixed at least 24h in zinc-formalin. Embedding and cutting of livers and slides preparation, staining and acquisition were performed by Centro di Imaging Sperimentale facility in San Raffaele hospital. Briefly, formalin-fixed paraffin-embedded consecutive sections (4µm) were dewaxed and hydrate through graded decrease alcohol series and stained for histology or immunohistochemical characterization (IHC). Slides were immunostained with Automatic Leica BOND RX system (Leica Microsystems GmbH, Wetzlar, Germany). First, tissues were deparaffinized and pre-treated with the Epitope Retrieval Solution (ER1 Citrate Buffer). Anti-GFP primary antibody (Invitrogen, A11122) was incubated 30 minutes at room temperature (1:1000) and was developed with Bond Polymer Refine Detection (Leica, DS9800). Slides were acquired with Aperio AT2 digital scanner at magnification of 20X (Leica Biosystems) and analysed with Imagescope (Leica Biosystem).

### Factor VIII (FVIII) Assays

The concentration of human FVIII was determined in mouse plasma by an enzyme-linked immunosorbent assay (ELISA) specific for human FVIII antigen. Microtiter plates were coated with anti-hFVlll binding Ab (Green Mountain Antibodies #GMA8016, 0.2µg/well in 0.1 M carbonate buffer, pH 9.6) overnight at 4°C and then blocked 1 hour at room temperature with blocking buffer (PBS 0.05% Tween-20, 1M NaCl, 10% heat inactivated horse serum, Gibco).

Plasma samples are diluted as needed starting from 1:10 in blocking buffer, added to wells (100µL/well) and incubated 2 hours at 37°C. hFVIII was detected by adding detection Ab (Affinity Biologicals, F8C-EIC-D) 1 hour at 37°C, followed by 5-10 minutes incubation with 100µL/well of TMB substrate (Surmodics). Reaction was blocked with HCI 1N (50µL/well) and absorbance of each sample was determined spectrophotometrically at 450nm, using a Multiskan GO microplate reader (Thermo Fisher Scientific) and normalized to antigen standard curve (ReFACTO, Pfizer, from 25ng/mL to 0.39ng/mL serially diluted 1:2 in blocking buffer; dilution was corrected with 10% HemoA murine plasma). hFVIII activity in mouse plasma was measured using Coatest SP FVIII (Chromogenix) following manufacturer's instructions.

Anti-hFVIII antibodies (Abs) were measured in mouse plasma by ELISA. Microtiter plates were coated with ReFACTO (Pfizer, 0.1µg/well in 0.1 M carbonate buffer, pH 9.6) over night at 4°C and then blocked 1 hour at room temperature with blocking buffer (PBS 0.05% Tween-20, 10% heat inactivated horse serum, Gibco). Samples are heat inactivated for 30 minutes at 56°C, diluted as needed starting from 1:100 in blocking buffer, added to wells (100µL/well) and incubated 2 hours at 37°C on orbital shaker. Anti-hFVIII Abs were detected by adding detection Ab (goat anti-mouse IgG-HRP, Sigma, 1:10,000 in blocking buffer) 1 hour at 37°C on orbital shaker, followed by 5-10 minutes incubation with 100µL/well of TMB substrate (Surmodics). Reaction was blocked with HCI 1N (50µL/well) and absorbance of each sample was determined spectrophotometrically at 450nm, using a Multiskan GO microplate reader (Thermo Fisher Scientific) and normalized to standard curve. The standard curve is a pool of 7 different commercial anti-human FVIII Abs raised against different FVIII domains (Green Mountain Antibodies #GMA8002, #GMA8005, #GMA8008, #GMA8011, #GMA8015, #GMA8016, #QED10104) serially diluted 1:2 from 200ng/mL to 1.56ng/mL in blocking buffer; dilution was corrected with 1% HemoA murine plasma.

### Statistical Analysis

Statistical analyses were performed upon consulting with professional statisticians at the San Raffaele University Center for Statistics in the Biomedical Sciences (CUSSB). When normality assumptions were not met, non-parametric statistical tests were performed. Two-tailed Mann-Whitney test was performed to compare two independent groups. Inferential techniques were applied in presence of adequate sample sizes (n ≥ 5), otherwise only descriptive statistics are reported.

### Example 1: GP64-LV Efficiently Transduce Mouse LSEC In Vivo

First, the *in vivo* administration of GP64-LV to mice was evaluated. GP64-LV or VSV.G-LV were produced expressing GFP under the control of the ubiquitously expressed phospho glycerate kinase (PGK) promoter, or human factor IX (FIX) under the control of the previously described hepatocyte-specific expression cassette (Milani *et al.* (2019)). GP64-LV.GFP or VSV.G-LV.GFP were administered i.v. to wild-type (wt) C57BL/6 adult mice at increasing doses and LV copies per cell (vector copy number, VCN) and GFP-positive tissue area in the liver were determined 1 week after LV administration. An LV-dose dependent increase of GFP marking was observed (Figure 1A). Similar GFP-positive tissue area was found in GP64-LV and VSV.G-LV treated mice. The distribution of the GFP signal among the main liver cell types was then analysed (with LSECs identified using the general endothelial cell marker, CD31), and it was observed that, at increasing doses, VSV.G-LV transduced more hepatocytes at the expense of KC, consistent with previous reports (Milani *et al.* (2019)). On the other hand, high doses of GP64-LV resulted in increased transduction of LSEC rather than hepatocytes, paralleled by a relative decrease in KC transduction **(****Figure 1B****).**

### Example 2: CD47hi GP64-LV Improve Transduction of Mouse LSEC In Vivo

Increasing doses of GP64-LV.FIX or CD47hi GP64-LV were then administered to adult NOD mice, which harbour a polymorphism in the CD47 receptor allowing cross-recognition of the human ligand, present on the LV particles (Takenata et al. (2007), Nat. Immunol., 8(12):1313-1323, doi: https://doi.org/10.1038/ni1527; and Milani *et al.* (2019)). VCN was determined from the main liver cell types upon FACS-sorting of liver cell subpopulations at the end of the experiment (3 months post LV administration; LSECs sorted using the general endothelial cell marker, CD31). No increase in the VCN of hepatocytes was observed, by using CD47hi GP64-LV at the highest dose. In contrast, high VCN was found in LSEC, reaching >10, which was even higher than that observed in GP64-LV.FIX treated NOD mice **(****Figure 1C****).**

These data confirm a high efficiency of gene transfer to LSEC *in vivo* mediated by GP64-LV systemic administration, further enhanced by combining CD47hi and GP64-pseudotyped LV.

### Example 3. GP64-LV Transduce Primary Human LSEC More Efficiently than VSV.G-LV

The transduction of primary human LSEC was then compared, by GP64-LV or VSV.G-LV expressing GFP, or a human codon-optimized FVIII transgene (Milani *et al.* (2022)), under the control of its endogenous endothelial-specific promoter, as previously described (Merlin *et al.* (2019)). Primary human LSEC were transduced ex *vivo* at different multiplicity of infection (MOI) with GFP or FVIII expressing vectors. Notably, transduction efficiency of these LSEC was similar or even higher by GP64-LV than VSV.G-LV, particularly at low MOI and also FVIII production measured in the cell supernatant was higher in GP64-LV transduced cells than in VSV.G-LV transduced cells **(****Figure 2****).**

These data show higher efficiency of transduction of primary human LSEC by GP64-LV compared to VSV.G-LV. This is in line with the mouse biodistribution data shown above and suggests that highly efficient gene transfer by GP64-LV is specifically achieved in LSEC.

### Example 4: GP64-LV Show Preferential Transduction of Liver Endothelial Cells over Endothelial Cells from Other Organs/Tissues

It was then investigated whether GP64-LV showed better tropism than VSV.G-LV for endothelial cells in general. The same dose of GP64-LV.GFP or VSV.G-LV.GFP were administered i.v. to adult C57BL/6 mice and the GFP expression in different organs was analysed by immunohistochemistry 10 days post administration. The majority of GFP signal was found to come from the liver and the spleen. Notably, except for in the liver, almost no endothelial cells were found to be GFP positive in other organs for either vector (endothelial cells were identified by their morphology), suggesting preferential transduction specifically of liver endothelial cells (of which the majority are LSECs as described hereinbefore), rather than the general microvascular endothelium **(****Figure 3****).**

### Example 5: GP64-LV Achieve Therapeutically Relevant FVIII Expression Mediated by the Endogenous Promoter in Haemophilia A Mice

The marked tropism for LSEC by GP64-LV prompted evaluation of *in vivo* FVIII gene transfer in haemophilia A mice. Adult immune tolerant haemophilia A mice (F8 knock out, KO, expressing a human mutated FVIII, R593C; Bril et al. (2006) Thromb. Haemost. 95(2):341-347, doi: https://doi.org/10.1160/th05-08-0559) were therefore treated by i.v. delivery of the same dose of GP64-LV or VSV.G-LV expressing the codon-optimized human FVIII under the control of the F8 promoter (Merlin *et al.* (2019)). Circulating human FVIII expression and activity was monitored longitudinally in treated mice. Interestingly, treatment with GP64-LV.FVIII allowed reconstituting about 10-20% of normal FVIII antigen and activity, while FVIII was undetectable in mice treated with VSV.G-LV.FVIII at this LV dose (Figures 4A-B). Anti-human FVIII Abs were lower in GP64-LV treated mice and did not neutralize FVIII activity **(****Figure 4C****).** Mice were then subjected to a haemostasis challenge to test phenotypic correction. All mice treated with GP64-LV survived, as well as wild-type mice, indicating haemostasis restoration in the former, while all control untreated mice, except for one, died. VSV.G-LV treated mice were not challenged since they did not display detectable amounts of human FVIII in the circulation **(****Figure 4D****).** Interestingly, VCN in LSEC was higher in GP64-LV than VSV.G-LV treated mice **(****Figure 4E****).** Moreover, LV transgene mRNA was detected only in LSEC of GP64-LV but not in VSV.G-LV treated mice, in line with the data of FVIII protein. No LV transgene mRNA was detected in purified hepatocytes, confirming endothelial-cell specific expression of the F8 promoter **(****Figure 4F****).**

These data confirm higher efficiency of gene transfer into LSEC by GP64-LV compared to VSV.G-LV and highlight the potential use of GP64-LV for *in vivo* LSEC-mediated haemophilia A gene therapy.

## Claims

1. A method of transducing liver endothelial cells with a nucleic acid, wherein the nucleic acid is transduced by means of a lentiviral vector particle pseudotyped with the baculovirus-derived GP64 envelope protein and comprising said nucleic acid.

2. The method of claim 1, wherein the nucleic acid comprises an endothelial cell-specific promoter and/or a liver-specific promoter operably linked to a coding sequence, in particular an endothelial cell-specific promoter, such as an LSEC-specific promoter,
optionally wherein the promoter is selected from: the long or short form endogenous human factor VIII (F8) promoter; the long or short form Stabilin2 promoter; the vascular endothelial cadherin (VEC) promoter; the intercellular adhesion molecule 2 (ICAM-2) promoter; the Tie2 promoter; or the flk-1 promoter.

3. The method of any one of claim 1 or claim 2, wherein the nucleic acid comprises a coding sequence for a gene for liver regeneration, or a gene defective or deficient in a liver disease or haemophilia, such as a coagulation and/or clotting factor,
optionally wherein the nucleic acid comprises factor VIII coding sequence,
optionally wherein the factor VIII coding sequence is a human codon-optimised factor VIII transgene.

4. The method of any one of claims 1 to 3, wherein the method is performed *in vitro, in vivo* or ex *vivo,* in particular *in vivo.*

5. A method of treating a subject in need of liver regeneration or suffering from a liver disease or haemophilia, the method comprising administering to the subject a lentiviral vector particle pseudotyped with the baculovirus-derived GP64 envelope protein and comprising a nucleic acid, said nucleic acid comprising a coding sequence for a gene for liver regeneration, or a gene defective or deficient in a liver disease or haemophilia.

6. The method of claim 5, wherein the nucleic acid comprises a coding sequence for a gene defective or deficient in haemophilia, such as haemophilia A, and
optionally wherein the gene defective or deficient in haemophilia encodes for a coagulation and/or clotting factor.

7. The method of claim 5 or claim 6, wherein the nucleic acid comprises a factor VIII coding sequence, optionally wherein the factor VIII coding sequence is a human codon-optimised factor VIII transgene, and/or
wherein the nucleic acid comprises a liver-specific promoter operably linked to the coding sequence, optionally an endothelial cell-specific promoter, such as an LSEC-specific promoter,
optionally wherein the promoter is selected from: the long or short form endogenous human factor VIII (F8) promoter; the long or short form Stabilin2 promoter; the vascular endothelial cadherin (VEC) promoter; the intercellular adhesion molecule 2 (ICAM-2) promoter; the Tie2 promoter; or the flk-1 promoter.

8. The method of any one of claims 5 to 7, wherein the lentiviral vector particle transduces liver endothelial cells in the subject,
optionally wherein the liver endothelial cells are liver sinusoidal endothelial cells (LSECs).

9. A lentiviral vector particle pseudotyped with the baculovirus-derived GP64 envelope protein for use in the transduction of liver endothelial cells with a nucleic acid *in vivo.*

10. The lentivirus vector particle for use of claim 9, wherein the nucleic acid comprises a liver-specific promoter operably linked to a coding sequence, optionally an endothelial cell-specific promoter, such as an LSEC-specific promoter,
optionally wherein the promoter is selected from: the long or short form endogenous human factor VIII (F8) promoter; the long or short form Stabilin2 promoter; the vascular endothelial cadherin (VEC) promoter; the intercellular adhesion molecule 2 (ICAM-2) promoter; the Tie2 promoter; or the flk-1 promoter.

11. The lentivirus vector particle for use of claim 9 or claim 10, wherein the transduction is for liver regeneration, or for the treatment of a liver disease or haemophilia, such as haemophilia A, and
optionally wherein the nucleic acid comprises a coding sequence for a gene for liver regeneration, or a gene defective or deficient in a liver disease or haemophilia, such as a coagulation and/or clotting factor, and/or
optionally wherein the nucleic acid comprises a factor VIII coding sequence,
optionally wherein the factor VIII coding sequence is a human codon-optimised factor VIII transgene.

12. A lentivirus vector particle pseudotyped with the baculovirus-derived GP64 envelope protein and comprising a nucleic acid, said nucleic acid comprising the long or short form endogenous human factor VIII (F8) promoter operably linked to a coding sequence.

13. The lentivirus vector particle of claim 12, wherein the nucleic acid further comprises a coding sequence for a gene for liver regeneration, or a gene defective or deficient in a liver disease or haemophilia, such as a coagulation and/or clotting factor,
optionally wherein the nucleic acid comprises a factor VIII coding sequence,
optionally wherein the factor VIII coding sequence is a human codon-optimised factor VIII transgene.

14. The method, lentivirus vector particle for use or lentivirus vector particle of any preceding claim, wherein the liver endothelial cells are liver sinusoidal endothelial cells (LSECs) and/or LSECs are transduced.

15. The method, lentivirus vector particle for use or lentivirus vector particle of any preceding claim, wherein the lentiviral vector particle further comprises high levels of CD47, or a functional fragment thereof, on its surface.
